(19) Europäisches Patentamt
European Patent Office
Office européen des brevets

(11) EP 2 114 481 B1

(12) # EUROPÄISCHE PATENTSCHRIFT

(45) Veröffentlichungstag und Bekanntmachung des Hinweises auf die Patenterteilung:
**30.09.2015 Patentblatt 2015/40**

(21) Anmeldenummer: **08706826.8**

(22) Anmeldetag: **30.01.2008**

(51) Int Cl.:
***A61L 31/14*** *(2006.01)* ***A61L 31/10*** *(2006.01)*
***A61L 31/02*** *(2006.01)* ***A61L 31/16*** *(2006.01)*

(86) Internationale Anmeldenummer:
**PCT/DE2008/000160**

(87) Internationale Veröffentlichungsnummer:
**WO 2008/092435 (07.08.2008 Gazette 2008/32)**

(54) **BIOLOGISCH ABBAUBARE GEFÄSSSTÜTZE**

BIODEGRADABLE VASCULAR SUPPORT

ENDOPROTHÈSE VASCULAIRE BIODÉGRADABLE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **30.01.2007 DE 102007005474**
**24.07.2007 DE 102007034350**

(43) Veröffentlichungstag der Anmeldung:
**11.11.2009 Patentblatt 2009/46**

(73) Patentinhaber: **Hemoteq AG**
**52146 Würselen (DE)**

(72) Erfinder:
- **HOFFMANN, Erika**
**52249 Eschweiler (DE)**
- **HOFFMANN, Michael**
**52249 Eschweiler (DE)**
- **HORRES, Roland**
**52223 Stolberg (DE)**

(74) Vertreter: **Arth, Hans-Lothar et al**
**ABK Patent Attorneys**
**Jasminweg 9**
**14052 Berlin (DE)**

(56) Entgegenhaltungen:
**EP-A- 1 891 992** **EP-A- 1 891 996**
**WO-A-2006/060033** **WO-A-2006/108065**
**WO-A-2006/116989** **WO-A-2007/005806**
**WO-A-2008/076582** **DE-A1-102005 018 356**
**US-A1- 2005 209 680** **US-A1- 2006 271 168**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 114 481 B1

## Beschreibung

Die vorliegende Erfindung betrifft biologisch abbaubare Gefäßstützen, welche aus einem inneren biodegradierbaren metallischen Gerüst und einer äußeren polymeren Beschichtung bestehen. Die bioabbaubare Beschichtung besteht bevorzugt aus biodegradierbaren Polymeren und kann zudem mindestens eine pharmakologisch aktive Substanz wie beispielsweise einen antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff enthalten.

Die Implantation von Stents ist heutzutage ein gängiger chirurgischer Eingriff zur Behandlung von Stenosen. Neuere Untersuchungen haben gezeigt, dass Gefäßverengungen jedoch nicht dauerhaft durch eine Endoprothese, insbesondere einen Stent aufgeweitet werden müssen. Es genügt, das Gewebe zeitweise durch eine Endoprothese aufzuweiten, da sich das Gewebe im Bereich der Gefäßverengung bei Anwesenheit einer Stützprothese regenerieren kann und dann auch ohne Unterstützung durch beispielsweise einen Stent in der aufgeweiteten Form verbleibt. Dies bedeutet, dass nach einer gewissen Zeit der Unterstützung des Gewebes durch eine Prothese, die Prothese ihre Wirkung weitgehend verliert, da das regenerierte Gewebe selbständig wieder in der Lage ist, den normalen Gefäßdurchmesser aufrecht zu erhalten, so dass nach Entfernung der Prothese keine erneute Gefäßverengung eintreten würde.

Ein bioresorbierbarer Metallstent aus überwiegend Magnesium ist in dem europäischen Patent EP 1 419 793 B1 offenbart. Die deutsche Offenlegungsschrift DE 102 07 161 A1 beschreibt Stents aus Magnesiumlegierungen und Zinklegierungen. Bioresorbierbare Stents aus Magnesium, Calcium, Titan, Zirkon, Niob, Tantal, Zink oder Silizium oder aus Legierungen oder Mischungen der vorgenannten Stoffe werden in der deutschen Offenlegungsschrift DE 198 56 983 A1 offenbart. Explizite Beispiele werden zu Stents aus einer Zink-Calcium-Legierung gegeben.

Weitere bioresorbierbare Metallstents aus Magnesium, Titan, Zirkon, Niob, Tantal, Zink und/oder Silizium als Komponente A und Lithium, Natrium, Kalium, Calcium, Mangan und/oder Eisen als Komponente B werden in der europäischen Patentanmeldung EP 0 966 979 A2 beschrieben. Explizite Beispiele werden zu Stents aus einer Zink-Titan-Legierung mit einem Titangewichtsanteil von 0,1 bis 1% und einer Zink-Calcium-Legierung mit einem Gewichtsverhältnis von Zink zu Calcium von 21:1 offenbart.

WO2007005806 beschreibt Stents mit einem inneren bioabsorbierbarem MetallGerüst und bioabbaubarer, poröser Polymer-Beschichtung, wobei die Beschichtung langsamer erodierbar ist bzw. eine längere Halbwertszeit besitzt als das Gerüst.

Diese Stents haben zum einen den Nachteil, dass sie sich zu schnell und zudem unkontrolliert auflösen und manche bereits nach 2 Wochen zerfallen sind.

Ein weiterer Nachteil dieser Stents sind die durch das Material vorgegebene notwendige Starre der Segmente, die sich im Vergleich mit den gängigen Stentmaterialien wie medizinischer Edelstahl Nitinol, Cobalt-Chrom-Stents im Design mit breiteren als auch dickeren Stentstreben äussert. Damit ergibt sich einerseits eine grössere Kontaktoberfläche zur Umgebung, andererseits reicht der Stent weiter in das Lumen hinein und kann den Blutfluss beeinflussen. Ebenso ist dadurch das Einwachsen in die Gefässwand aufgrund der grösseren zu überdeckenden Oberfläche verzögert.

Da zudem der Auflösungsprozess beginnt, bevor der Stent in die Gefäßwand eingewachsen ist, können sich Bruchstücke lösen, durch die Blutbahn transportiert werden und einen Herzinfarkt auslösen.

Ein weiterer Nachteil der beschriebenen bioresorbierbaren Metallstents, liegt in der nur sehr beschränkten Integrationsmöglichkeit eines pharmakologischen Wirkstoffes in das Metallgerüst, der bei dem Abbau des Stents freigesetzt wird.

Aufgabe der vorliegenden Erfindung ist es nun, eine Gefäßstütze bereitzustellen, welche ihre Stützfunktion nur so lange ausübt, bis das regenerierte Gewebe wieder selber in der Lage ist, diese Funktion zu übernehmen und die Nachteile des Standes der Technik vermieden werden.

Diese Aufgabe wird durch die technische Lehre des unabhängigen ersten Anspruches der vorliegenden Anmeldung gelöst. Weitere vorteilhafte Ausgestaltungen der Erfindung ergeben sich aus den abhängigen Ansprüchen, der Beschreibung sowie den Beispielen.

Die vorliegende Erfindung betrifft biologisch abbaubare Gefäßstützen insbesondere Stents, welche aus einem inneren zumindest ein Metall enthaltenden, bioresorbierbaren Gerüst bestehen, welches von einer polymeren biologisch abbaubaren Beschichtung umgeben ist.

Die polymere Schicht reduziert sich auf die Stentstreben selbst oder kann den gesamten Hohlkörper wie ein Strumpf wahlweise auf der abluminalen als auch der luminalen Seite des Stentkörpers einhüllen bzw. die freien Zwischenräume des Stentkörpers derart ausfüllt, dass die Hülle in einer Ebene mit den ebenfalls umhüllten Stentstreben liegt. Die Beschichtungsformen sind in sinnvoller Weise kombinierbar.

Erfindungsgemäß besteht das innere Gerüst der Gefäßstütze aus einem Metall, einer Metalllegierung, Metalloxid, Metallsalz, Metallcarbid, Metallnitrid oder eine Mischung der vorgenannten Stoffe.

Insbesondere bevorzugt ist, wenn das innere Gerüst aus einer Metalllegierung besteht, welche bis zu 30 Gew.-%, bevorzugt bis zu 20 Gew.-% und insbesondere bevorzugt nur bis zu 10 Gew.-% an Metalloxiden, Metallsalzen,

Metallcarbiden und/oder Metallnitriden enthält. Ferner können bis zu 1 Gew.-% an weiteren Bestandteilen wie beispielsweise Kohlenstoff, Stickstoff, Sauerstoff, Verunreinigungen, Nichtmetalle oder organische Substanzen in der Zusammensetzung oder in der Legierung enthalten sein.

Das innere metallische Gerüst hat zudem die Eigenschaft, dass es sich schneller auflöst, als die polymere Außenhülle, d.h. die innere Struktur der Gefäßstütze wird biologisch schneller unter physiologischen Bedingungen abgebaut als die polymere Beschichtung. Bei der Verwendung verschiedener bioabbaubarer Polymere auf einem Stent besteht zudem die Möglichkeit zeitlich unterschiedlich schnell abbaubare Polymere zu verwenden. Erfindungsgemäss löst sich die luminale Beschichtung langsamer als die abluminale Stentbeschichtung.

Beispielsweise wird dadurch der Stentabbau von Seiten des Blutflusses verzögert. Ein weiterer Vorteil besteht in der Stabilisierung des Stentkörpers, so dass sich nicht vorzeitig Bruchstücke lösen können. Eine vollständige vollflächige Einhüllung des Stentkörpers auf der Innenseite kann diese Effekte noch weiter regulieren. Vorzugsweise wird die Metalllegierung innerhalb der polymeren Hülle in die entsprechenden Metallsalze überführt, welche durch die polymere Beschichtung austreten können.

Geeignete metallische innere Gerüste der Gefäßstütze sind aus solchen metallischen Werkstoffen, welche eine Potentialdifferenz von mindestens -0,48 eV, bevorzugt von mindestens -0,53 eV, weiter bevorzugt von mindestens -0,58 eV und insbesondere bevorzugt von mindestens -0,63 eV im Vergleich zur Kalomelelektrode aufweisen oder eine Potentialdifferenz im Bereich von -0,3 bis -2,5 eV, bevorzugt-0,4 bis -1,5 eV, weiter bevorzugt von -0,45 bis -1,25 eV und insbesondere bevorzugt von -0,5 bis -1,0 eV im Vergleich zur Kalomelelektrode besitzen.

Um Potentialdifferenzen messtechnisch zu erfassen, bedient, man sich einer elektrochemischen Anordnung zweier Halbzellen. Da die Potentialdifferenz reproduzierbar bestimmt werden sollte, ist ein Bezugspunkt, der sich während der Messung nicht verändern sollte, notwendig.

Für diesen Zweck benutzt man in der Regel "Elektroden zweiter Art". Dabei handelt es sich um Metallelektroden, die mit ihren unlöslichen Salzen bedeckt sind und von einer Salzlösung höherer Konzentration umspült werden. Dazu gehört zum Beispiel die Kalomelelektrode (engl.: Saturated Calomel Electrode, SCE). Der Name "Kalomel" leitet sich vom Trivialnamen des schwerlöslichen Quecksilber(I)-Chlorids ab.

Die Kalomelelektrode (wie auch einige andere Metall/Metallsalz-Elektroden) haben sich in der Praxis als Bezugselektroden bewährt. Eine praktische Anwendung ist zum Beispiel die Messung einer Potentialdifferenz in Lösung mit Hilfe der Kalomelektrode. Eine solche Messung kann auch zur Bestimmung eines geeigneten Metalls bzw. einer geeigneten Metalllegierung verwendet werden.

Die Potentialdifferenz wird üblicherweise durch die bekannte Nernst-Gleichung beschrieben:

$$E_{\frac{Hg_2^{2+}}{Hg}} = E^0_{\frac{Hg_2^{2+}}{Hg}} + \frac{0{,}059V}{2} \cdot \lg c_{Hg_2^{2+}}$$

Wie man leicht sieht, ist das Potenzial E nur von der Konzentration des schwerlöslichen Quecksilbersalzes abhängig. Hält man nun die Anionenkonzentration, also die Konzentration des Gegenions konstant, so bleibt auch das Potential E konstant. Man kann dies erreichen, in dem man die AnionenKonzentration sehr groß wählt. Der Aufbau der Kalomelelektrode ist schematisch in Fig. 1 gezeigt.

Die Kalomelelektrode besteht aus Quecksilber, der eigentlichen Elektrode, das mit festem $Hg_2Cl_2$ überschichtet ist und in eine gesättigte KCl-Lösung (hohe Konzentration von $Cl^-$-Ionen) taucht. Die Salzbrücke wird für genaue Messungen zur Ausschaltung von Diffusionspotentialen benutzt. Tabellen, die Messwerte enthalten, die mit diesem Aufbau erhalten werden, müssen immer gegen diesen Bezugspunkt (Kalomelektrode) tabelliert werden. Dabei muss auch die Konzentration der KCl-Lösung angegeben werden.

Somit eignet sich die Kalomelelektrode als Elektrode zweiter Art sehr gut als Bezugselektrode für Potentialmessungen. Die Kalomelelektrode wird auch in der vorliegenden Erfindung als Bezugselektrode gewählt.

Der oben skizzierte Aufbau kann nun dazu benutzt werden, um geeignete Materialien auszuwählen, die unedler als Kalomel sind, d.h. ein Vergleichspotenzial von 0,3 bis 2,5 eV, bevorzugt von 0,35 bis 2,2 eV, weiter bevorzugt von 0,4 bis 1,8 eV, weiter bevorzugt von 0,45 bis 1,4 eV, weiter bevorzugt von 0,48 bis 1,2 eV, weiter bevorzugt von 0,50 bis 1,0 eV, weiter bevorzugt von 0,50 bis 0,9 eV, weiter bevorzugt von 0,50 bis 0,80 eV und insbesondere bevorzugt von 0,50 bis 0,70 eV (angegeben als Beträge, d.h. mit positivem Vorzeichen) im Vergleich zur Kalomelelektrode besitzen.

Besonders bevorzugt ist, wenn das innere Gerüst aus einer Legierung besteht, welche Magnesium, Calcium, Mangan, Eisen, Zink, Silizium, Yttrium, Zirconium und/oder Gadolinium enthält und weiter bevorzugt wenn in dieser Legierung Magnesium, Calcium, Mangan, Eisen, Zink, Silizium, Yttrium, Zirconium oder Gadolinium den größen Gewichtsanteil angegeben in Gew.-% ausmacht.

Um zu vermeiden, dass sich das metallische Gerüst zu schnell auflöst und in Bruchstücke zerfällt, welche durch

die Blutbahn weggespült werden können und einen Herzinfarkt auslösen können, wird das innere bioresorbierbare Gerüst aus Metall, Metallsalz, Metalloxid und/oder Metalllegierung mit einer polymeren Beschichtung umgeben, die die Stentstreben einhüllt oder, wie bereits erwähnt, den gesamten zylindrischen Stentkörper bedeckt.

Erfindungsgemäß ist die polymere Beschichtung derart ausgestaltet, dass sich das innere metallische Gerüst in der Beschichtung auflösen kann und die Metallionen durch die Beschichtung in das umliegende Gewebe austreten können. Die polymere Beschichtung ist somit porös oder mit Kanälen oder Löchern versehen und insgesamt so ausgestaltet, dass einen Durchtritt von Ionen (Anionen als auch Kationen) stattfinden kann.

Die polymere Beschichtung kann erfindungsgemäß in Form einer ionendurchlässigen Membran oder mit Nano- bis Mikroporen versehen sein, welche den Wasserdurchtritt als auch den Ionenaustritt ermöglichen.

Derartige poröse oder mit Kanälen oder Löchern versehene polymere Beschichtungen erhält man, indem man entweder eine polymere Beschichtung auf den Stent aufbringt, welche zu einer durchlässigen Polymerschicht führt oder indem die polymere Beschichtung nach der Aufbringung durchlässig gemacht wird. Unter dem Begriff "durchlässig" soll verstanden werden, dass die polymere Beschichtung porös ist oder Kanäle, Poren oder Löcher besitzt, welche den Wassereintritt und den Ionenaustritt ermöglichen.

Solche Beschichtungen können zum einen erhalten werden durch Polymere, welche von sich aus auf der Stentoberfläche zu einer porösen Beschichtung führen oder durch eine Lösung von Oligomeren und/oder Polymeren, welche auf die Stentoberfläche aufgetragen wird und wobei die Oligomeren und/oder Polymeren nach Auftragung einer weiteren Quervernetzung (beispielsweise durch Glutaraldehyd oder andere Dialdehyde) unterzogen werden und danach nicht quervernetzte Oligomere und Polymere von der Beschichtung vorzugsweise mittels eines Lösungsmittels abgewaschen werden oder durch die Verwendung von autopolymerisierbaren Substanzen wie z.B. ungesättigten Fettsäuren und Derivate ungesättigter Fettsäuren, wobei die nicht polymerisierten Substanzen von der Stentoberfläche vorzugsweise durch ein Lösungsmittel abgewaschen werden. Weitere Möglichkeiten zur Erzeugung einer durchlässigen Polymerbeschichtung auf dem Stent sind die Aufbringung einer relativ unflexiblen oder starren bzw. brüchigen Polymerschicht, welche bei der Expansion des Stents aufbricht und Risse bildet und somit vorzugsweise erst nach der Inflation des Stents durchlässig wird. Ferner kann der polymeren Beschichtungslösung eine oder mehrere Substanzen zugesetzt werden, nach Aufbringung der Beschichtung auf dem Stent wieder ausgewaschen werden können und danach eine durchlässige Struktur zurücklassen. Bevorzugt ist hier die Zugabe von Salzen in Form von Pulver, Partikeln oder auch in gelöster Form. Nach Ausbildung der polymeren Beschichtung können die Salze vorzugsweise durch Wasser aus der polymeren Schicht ausgewaschen werden und hinterlassen eine poröse Struktur. Natürlich muss das Auswaschen der Salze nicht vor Stentimplantation erfolgen. Der Stent mit Polymerbeschichtung samt enthaltener physiologisch verträglicher Salze kann auch in noch nicht durchlässiger Form implantiert werden und das Auswaschen der Salze erfolgt dann auf natürlichem Wege im Blutstrom, wobei dann erst eine durchlässige Beschichtung nach der Implantation erhalten wird, wenn das Auswasschen der physiologisch verträglichen Salze wie z.B. NaCl, NaBr, NaI, $NaSO_4$, KCl, $NaHCO_3$, oder anderer dem Fachmann bekannte physiologisch verträgliche Salze, aus der polymeren Beschichtung herausgelöst worden sind.

Abschließend besteht natürlich auch die Möglichkeit, eine nicht durchlässige Polymerbeschichtung auf dem Stent zu erzeugen und diese danach durch chemische, mechanische, optiche oder andere Verfahren durchlässig zu machen. Beispielsweise kann durch den Einsatz von Basen oder Säuren die Polymerbeschichtung durchlässig gemacht werden als auch durch den Einsatz von Lasern oder durch mechanische Polierverfahren in Form von chemical polishing Verfahren oder Sandstrahlverfahren. Derartige Techniken sind dem Fachmann bekannt und sind natürlich auf die jeweilige Beschichtung, deren Dicke und Härte und dem verwendeten Polymeren anzupassen.

Durch diese Ausführungsform wird sichergestellt, dass zumindest anfangs ein Metallenthaltendes inneres Gerüst vorhanden ist, welches genügend Spreizkraft auf das Gefäß auswirken kann, um dieses offen zu halten und einen spontanen Recoil, d.h. ein nach der Dilatation spontanes Zusammenfallen des Gefäßes aufgrund geschädigter oder erschlaffter Gefäßmuskulatur zu verhindern. Da jedoch ein Gefäß seine Elastizität und Spannkraft nach einer gewissen Zeit wiedererlangen kann, ist somit ein Stent in Form eines Dauerimplantats, d.h. eines nicht oder nur extrem langsam biologisch abbaubaren Implantats nicht erforderlich, um das Gefäß dauerhaft offen zu halten.

Zudem besteht bei nicht biologisch abbaubaren Stents das Problem der Restenose bzw. der In-Stent-Restenose, wobei sich durch Überwucherung des Stent mit glatten Muskelzellen das Gefäß im Inneren des Stents wieder verengt bis verschließt. Hier besteht ferner das Problem, an eine Stelle, wo bereits ein nicht biologisch abbaubarer Stent gesetzt worden ist, einen weiteren Stent zu platzieren.

Desweiteren ist bei Verwendung von Wirkstoff freisetzenden Stents (DES) aus den bekannten nicht bioabbaubaren Metallstentmaterialien die Gefahr der "late thrombosis", die oft erst nach einem Jahr überraschend zu einem akuten Verschluss führen kann, gegeben. Die beunruhigenden Ergebnisse wurden im Sommer 2006 bekannt gemacht und als Ursache der gehäuft auftretenden späten Thrombose wurde die nach dieser Zeit aufgrund der Zellwachstumshemmung des Wirkstoffs immer noch nicht eingewachsene Oberfläche des Stents gemacht. Damit wurde und wird immer noch der mit dem DES eingetretene Benefit massiv in Frage gestellt.

Auch diese Nachteile vermeidet der erfindungsgemäße Stent, da er sich kontrolliert nach einer gewissen Zeit

vollständig auflöst. Die erfindungsgemäße Polymerhülle ermöglicht den biologischen Abbau des metallischen Innengerüsts, ohne dass die Gefahr besteht, dass Bruchstücke sich lösen können, da die Polymerhülle das innere Gerüst derart vollständig umgibt, dass größere oder auch kleinere Bruchstücke nicht durch die polymere Beschichtung hindurchtreten können. Ermöglicht ist jedoch der Durchtritt von Ionen und Salzen, welche sich aus dem metallischen Gerüst unter physiologischen Bedingungen bilden.

Solche Metallionen können inklusive ihrer Gegenanionen durch die polymere Beschichtung hindurchtreten bzw. aus den Nano- bis Mikroporen austreten.

Bei einer besonders bevorzugten Ausführungsform der vorliegenden Erfindung wird das innere metallische oder ein Metall enthaltende Gerüst schneller unter physiologischen Bedingungen abgebaut als die äußere polymere Hülle, so dass nach Degradation des Innengerüstes eine in der Gefäßwand eingewachsene leere Polymerhülle eine Zeitlang zurückbleibt, welche jedoch flexibel ist und keinen nennenswerten Druck auf die Gefäßwand mehr ausübt und sich sogar dem neuen Gefäßverlauf gut anpasst. Danach wird auch diese polymere Hülle biologisch abgebaut, so dass nach 2 bis 12 Monaten der biologisch abbaubare Stent sich vollständig aufgelöst hat. Somit ist erfindungsgemäß, dass sich die polymere Beschichtung langsamer auflöst als die metallische Innenstruktur und den Austritt von Salzen und Ionen ermöglicht, damit sich die innere Struktur auflösen kann und die Salze und Ionen von dem umliegenden Gewebe resorbiert werden können. Bei dieser besonders bevorzugten Ausführungsform der vorliegenden Erfindung ist der beschichtete Stent so konzipiert, dass der Stent in das Gewebe eingewachsen ist, bevor sich die bioresorbierbare Beschichtung beginnt aufzulösen. Die Auflösung des inneren Stentgerüstes kann bereits vor dem Einwachsen des Stents in das Gefäßgewebe erfolgen, wobei vorzugsweise das Einwachsen und die Auflösung des inneren Stentgerüstes weitgehend gleichzeitig verlaufen. Hingegen ist die Auflösungsgeschwindigkeit von innerer Stentstruktur im Vergleich zur darauf aufgebrachten Beschichtung wesentlich bei dieser besonders bevorzugten Ausführungsform. Vorzugsweise sollte die polymere Beschichtung zu maximal 15 Gew.-% bevorzugt maximal 10 Gew.-% und insbesondere bevorzugt zu maximal 5 Gew.-% aufgelöst sein, wenn der innere Stentkörper vollständig aufgelöst ist. Unter dem Begriff "polymere Beschichtung" sind nut die die polymere Beschichtung bildenden Beschandteile zu verstehen und keine Bestandteile der Beschichtung, welche nicht in polymerer Form gebunden sind wie beispielsweise Salzpartikel, welche durch den Blutstrom aus der Beschichtung ausgewaschen werden sollen. Anders beschrieben, sollte die Auflösungsgeschwindigkeit von innerem Stentgerüst zu polymerer Beschichtung mindestens 10 : 1, vorzugsweise 20 : 1, weiter bevorzugt 30 : 1, noch weiter bevorzugt 40 : 1 und insbesondere bevorzugt 50 : 1 betragen. Hierbei bedeutet ein Verhältnis von 20 : 1, dass zumindest 20 Gew.-% des inneren Stentgerüstes aufgelöst und durch die polymere Beschichtung abgegeben wurden, wenn maximal 1 Gew.-% der polymeren Beschichtung aufgelöst bzw. biologisch abgebaut worden ist.

Eine Möglichkeit die Auflösungskinetik eines erfindungsgemäßen unbeschichteten Metallstents zu bestimmen besteht darin, diesen in einem Schlauch zwischen zwei porösen Membranen oder Filterplatten zu platzieren und physiologische Kochsalzlösung, PBS-Puffer (Phosphatpuffer mit 14,24 g $NaH_2PO_4$, 2,72 g $K_2HPO_4$ und 9 g NaCl; pH 7,4; T=37°C) oder Blutserum durch den Schlauch zu leiten, vorzugsweise zudem mit einer ähnlichen Geschwindigkeit wie der Blutstrom im menschlichen Körper durch die Blutgefäße fließt.

Die Auflösungsgeschwindigkeit der polymeren Beschichtung kann bestimmt werden, indem man die polymere Beschichtung auf einen nicht bioresorbierbaren Stent, z.B. einen Edelstahlstent aufbringt und ebenfalls zwischen zwei Diaphragma in einem Schlauch platziert, durch den physiologische Kochsalzlösung, PBS-Puffer oder Blutserum geleitet wird.

Die Auflösung von Stentgerüst und Beschichtung kann optisch verfolgt und auch durch Gewichtsbestimmung quantitativ verfolgt werden.

Bei der erfindungsgemäßen Ausführungsform weist die polymere Beschichtung Löcher, Öffnungen und/oder Kanäle auf, welche den Salzaustritt bzw. den Ionenaustritt ermöglichen, jedoch nicht groß genug sind, dass Bruchstücke des metallischen Innengerüstes austreten können.

Diese Löcher, Öffnungen und/oder Kanäle verlaufen vorzugsweise senkrecht zur Mittelachse der einzelnen Stentstreben und sind zudem vorzugsweise nicht an den Enden der Stentstreben angebracht. Diese Löcher, Öffnungen und/oder Kanäle können mechanisch, chemisch, thermisch oder optisch in das Polymer eingebracht werden, beispielsweise durch mechanische Behandlung wie Sandstrahlung, durch chemische Verfahren wie Ätzung oder Oxidation, durch mechanisch-chemische Verfahren wie Polierverfahren, durch thermische Verfahren wie Schmelzen oder Einbrennen oder durch optische Verfahren wie Laserbehandlung.

Bei einer weiteren insbesondere bevorzugten Ausführungsform werden die Löcher, Öffnungen und/oder Kanäle mit einem pharmakologischen Wirkstoff gefüllt. Geeignete Wirkstoffe sind unten genannt. Der oder die in die Löcher, Öffnungen und/oder Kanäle einzubringende(n) Wirkstoff(e) können mit einem pharmakologisch verträglichen Träger vermischt sein, wie beispielsweise einem Salz, einem Kontrastmittel, einem Füllstoff, einem Oligomer, einer organischen Verbindungen wie beispielsweise Aminosäuren, Vitaminen, Kohlenhydraten, Fettsäuren, Ölen, Fetten, Wachsen, Proteinen, Peptiden, Nukleotiden oder einem Lösungsmittel.

Als pharmakologisch verträgliche Träger können beispielsweise Lactose, Stärke, Natrium-Carboxymethylstärke, Sorbitol, Sucrose, Magnesiumstearat, Dicalciumphosphat, Calciumsulfat, Talk, Mannitol, Ethylalkohol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Myristate wie Isopropylmyristat, Palmitate, Tributyl- und Triethylcitrate und deren Acetylderivate, Phthalate wie Dimethylphthalat oder Dibutylphthalat, Benzoesäurebenzylester, Triacetin, 2-Pyrrolidon, Borsäure, Magnesium-Aluminum-Silicate, natürliches Johannisbrot-kernmehl, Karaya, Guar, Tragacanth, Agar, Carrageenane, Cellulose, Cellulosederivate wie Methylcellulose, Natrium-Carboxymethylcellulose, Hydroxypropyl-methylcellulose, microkristalline Cellulose sowie Alginate, Tonerden und Bentonite, Polyethylenglycol als auch Wachse wie z.B. Bienenwachs, Carnaubawachs, Candelillawachs und dergleichen eingesetzt werden.

Weitere Trägerstoffe können Vitamine wie z.B. Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure sein.

Weitere geeignete Trägerstoffe sind Heparin, Heparansulfate, Chitosan, Chitin, Chondroitinsulfat, Collagen, Fibrin, Xanthone, Flavonoide, Terpenoide, Cellulose, Rayon, Peptide mit 50 bis 500 Aminosäuren, Nukleotide mit 20 bis 300 Basen sowie Saccharide mit 20 bis 400 Zuckerbausteinen, Fettsäuren, Fettsäureester, Fettsäurederivate, Ether, Lipide, Lipoide, Glyceride, Triglyceride, Glycolester, Glycerinester, und Öle wie z.B. Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle.

Als Aminosäuren eignen sich Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N,N,N-Trimethyllysin, 3-Methylhistidin, 5-Hydroxylysin, O-Phosphoserin, γ-Carboxyglutamat, ε-N-Acetyllysin, ω-N-Methylarginin, Citrullin, Ornithin.

Des weiteren sind die folgenden Fettsäuren sowie die Ester der folgenden Fettsäuren als Trägerstoffe geeignet: Eicosapentaensäure (EPA), Timnodonsäure, Docosahexaensäure (DHA), α-Linolensäure, γ-Linolensäure, Myristoleinsäure, Palmitoleinsäure, Petroselinsäure, Ölsäure, Vaccensäure, Gadoleinsäure, Gondoinsäure, Erucinsäure, Nervonsäure, Elaidinsäure, t-Vaccensäure, Linolsäure, γ-Linolsäure, Dihomo-γ-linolensäure, Arachidonsäure, α-Linolensäure, Stearidonsäure, DPA, Meadsäure, Stellaheptaensäure, Taxolsäure, Pinolensäure, Sciadonsäure, Taririnsäure, Santalbin- oder Ximeninsäure, Stearolinsäure, 6,9-Octadeceninsäure, Pyrulinsäure, Crepenynsäure, Heisterinsäure, ETYA, Laurinsäure, Myristinsäure, Palmitinsäure, Margarinsäure, Stearinsäure, Arachinsäure, Behensäure und Lignocerinsäure sowie Derivate und Mischungen der vorgenannten Fettsäuren.

Insbesondere bevorzugt ist jedoch, den mindestens einen antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff in einem Lösungsmittel zu lösen und weitgehend als reinen Wirkstoff in die Löcher, Öffnungen und/oder Kanäle in der polymeren Beschichtung einzubringen, was über ein Spritzenverfahren oder ein Pipettierverfahren erfolgen kann. Nach Verdunstung des Lösungsmittels bleibt der Wirkstoff in den Löchern, Öffnungen und/oder Kanälen zurück.

Als Lösungsmittel dienen die üblichen organischen Lösungsmittel wie Dimethylsulfoxid, Ether wie beispielsweise Dioxan, Tetrahydrofuran (THF), Petrolether, Diethylether, Methyl-tert.-butylether (MTDC), Ketone wie beispielsweise Aceton, Butanon oder Pentanon, Alkohole wie beispielsweise Methanol, Ethanol, Propanol, Isopropanol, Carbonsäuren wie beispielsweise Ameisensäure, Essigsäure, Propionsäure, Amide wie beispielsweise Dimethylformamid (DMF) oder Dimethylacetamid, aromatische Lösungsmittel wie beispielsweise Toluol, Benzol, Xylol, reine Kohlenwasserstofflösungsmittel wie beispielsweise Pentan, Hexan, Cyclohexan, halogenierte Lösungsmittel wie beispielsweise Chloroform, Methylenchlorid, Tetrachlorkohlenstoff, sowie Carbonsäureester wie beispielsweise Essigsäuremethyl- und Essigsäureethylester als auch Wasser, je nach Löslichkeit des Wirkstoffs.

Zudem ist insbesondere bevorzugt, den Wirkstoff in einem Kontrastmittel oder Kontrastmittelanalogon aufzunehmen und in dieser Form in die Löcher, Öffnungen und/oder Kanäle einzubringen.

Als Kontrastmittel oder Kontrastmittelanaloga können die üblichen Röntgenkontrastmittel (positive Kontrastmittel als auch negative Kontrastmittel) verwendet werden, wie sie für bildgebende Verfahren (Arthrographie, Röntgenaufnahmen, Computertomographie (CT), Kernspintomographie, Magnetresonanztomographie (MRT)) üblicherweise eingesetzt werden.

Kontrastmittel und/oder Kontrastmittelanaloga enthalten zumeist Barium, Iod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium bevorzugt als Ionen in gebundener und/oder komplexierter Form, wobei iodhaltige Kontrastmittel bevorzugt sind.

Als iodhaltige Kontrastmittel können folgende Beispiele genannt werden:

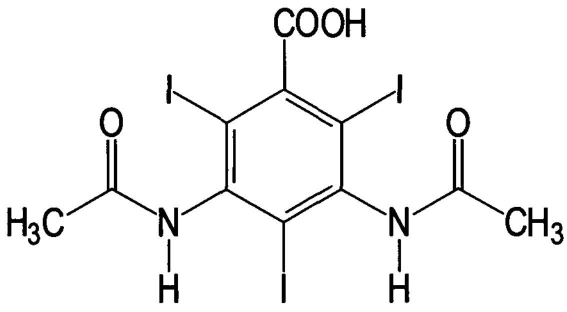

Amidotrizoesäure

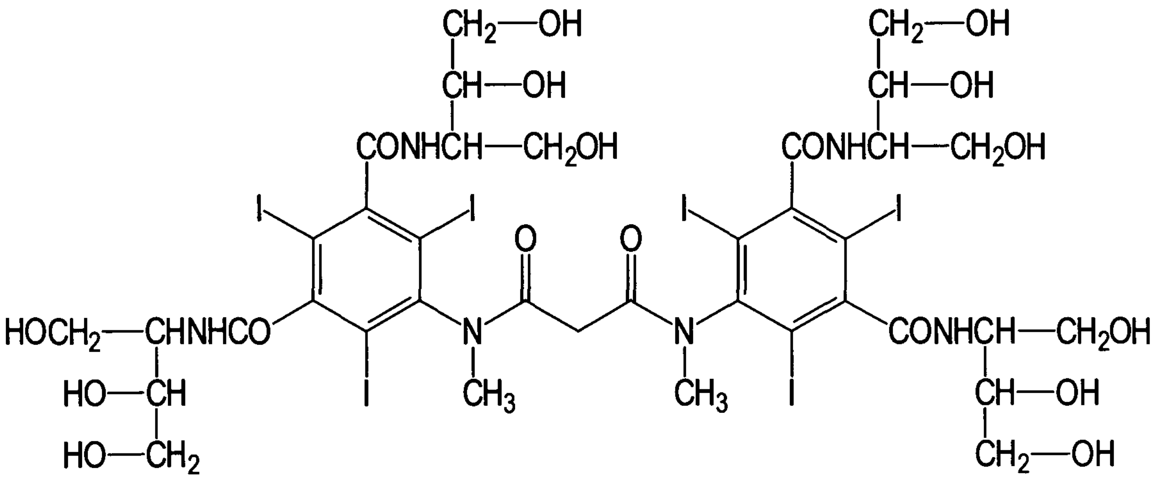

Iotrolan

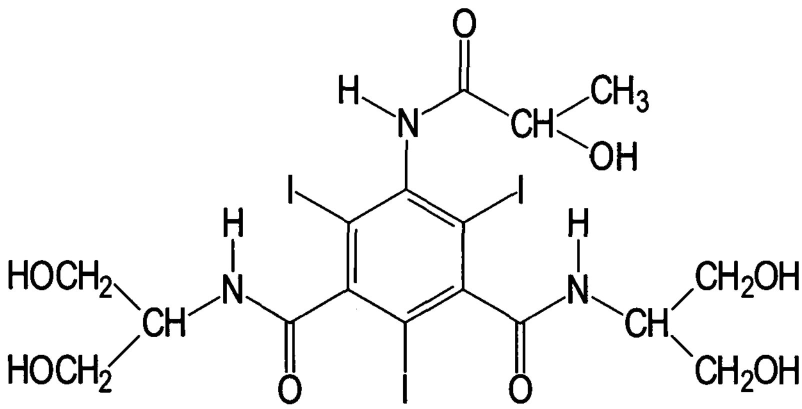

Iopamidol

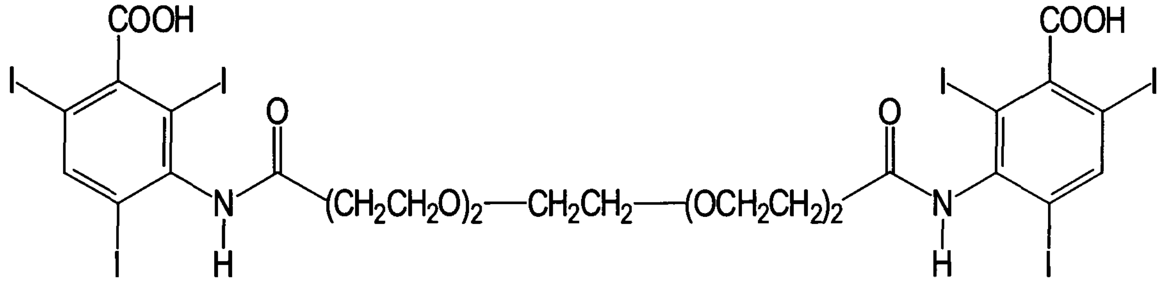

Iodoxaminsäure

Ein weiteres Beispiel ist Jod-Lipiodol®, ein iodiertes Oleum papaveris, ein Mohnöl. Unter den Handelsnamen Gastrografin® und Gastrolux® ist die Muttersubstanz der iodierten Kontrastmittel, das Amidotrizoat in Form von Natrium- und Megluminsalzen kommerziell erhältlich.

Auch Gadolinium-enthaltende oder superparamagnetische Eisenoxidpartikel sowie ferrimagnetische oder ferromagnetische Eisenpartikel wie beispielsweise Nanopartikel sind bevorzugt.

Eine weitere Klasse von bevorzugten Kontrastmitteln stellen die paramagnetischen Kontrastmittel dar, welche zumeist ein Lanthanoid enthalten.

Zu den paramagnetischen Substanzen, die über ungepaarte Elektronen verfügen, zählt z.B. das Gadolinium ($Gd^{3+}$), das insgesamt sieben ungepaarte Elektronen besitzt. Des weiteren gehören zu dieser Gruppe das Europium ($Eu^{2+}$, $Eu^{3+}$), Dysprosium ($Dy^{3+}$) und Holmium ($Ho^{3+}$). Diese Lanthanoide können auch in chelatisierter Form unter Verwendung von beispielsweise Hämoglobin, Chlorophyll, Polyazasäuren, Polycarbonsäuren und insbesondere EDTA, DTPA, DMSA, DMPS sowie DOTA als Chelatbildner eingesetzt werden.

Beispiele für Gadolinium-haltige Kontrastmittel sind Gadolinium-Diethylentriaminpentaessigsäure

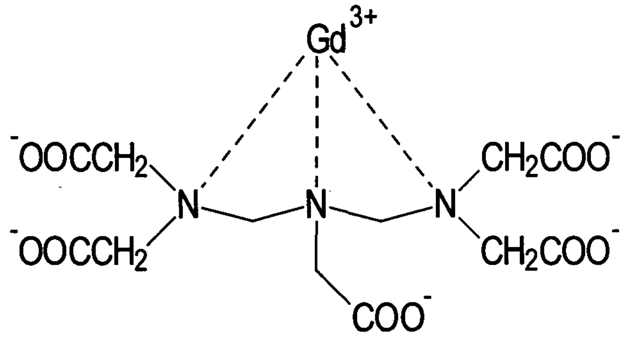

Gadopentetsäure (GaDPTA)

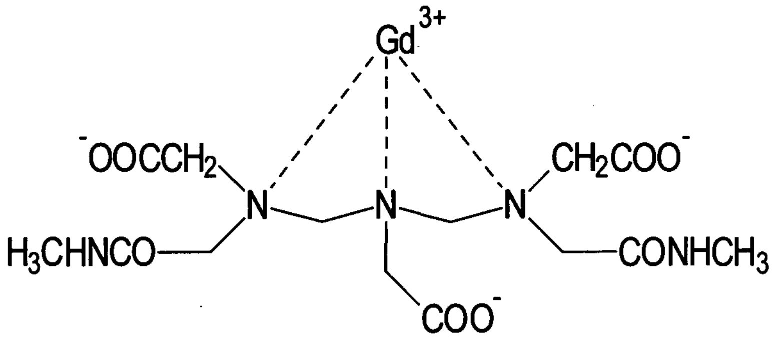

Gadodiamid

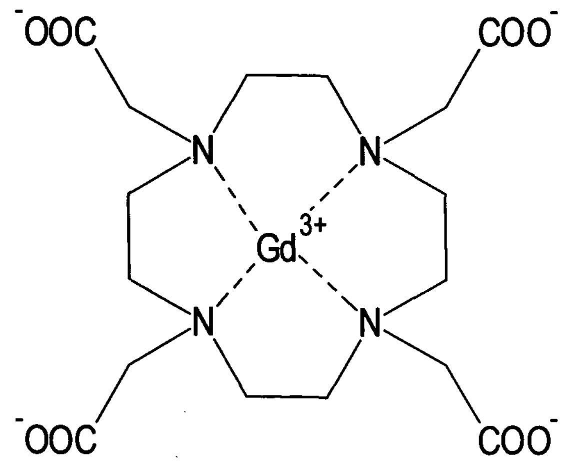

Meglumin-Gadoterat

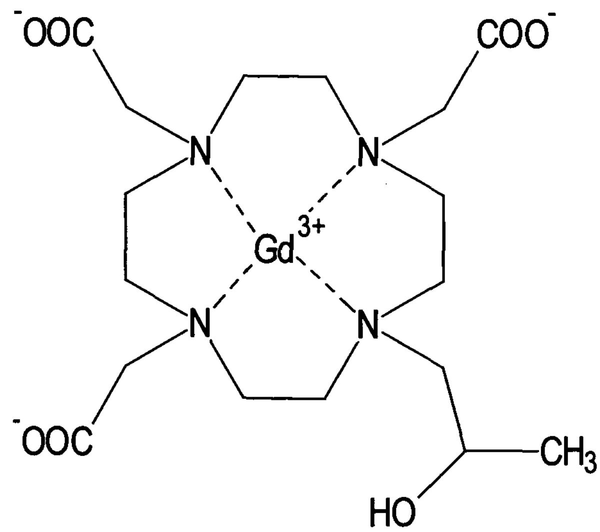

Gadoteridol

Zur Steigerung der Wirkstoffübertragung können bevorzugt sogenannte Transportvermittler eingesetzt werden, welche jedoch auch selber den Wirkstoff darstellen können. Von besonderem Interesse sind niedermolekulare chemische Verbindungen als Transportvermittler enthalten, die die Aufnahme von Wirkstoffen in die Gefäßwand beschleunigen bzw. erleichtern, so dass der vorhandene Wirkstoff bzw. die Wirkstoffkombination während des kurzzeitigen Kontaktes kontrolliert und in der vorgesehenen Dosierung übertragen werden kann.

Derartige Eigenschaften besitzen Substanzen, die entweder direkt mit der Lipiddoppelschicht der Zellmembran wechselwirken, mit Rezeptoren auf der Zellmembran interagieren, über Membrantransportproteine, die als Carrier oder als Kanal (Ionenpumpe) wirken, in das Zellinnere gelangen, wo sie das Membranpotential und damit die Membranpermeabilität der Zellen verändern. Dadurch wird die Aufnahme eines Wirkstoffs in die Zellen erleichtert bzw. beschleunigt.

Zu solcherart nutzbaren Verbindungen gehören beispielsweise die Vasodilatoren, zu denen körpereigene Substanzen wie die Kinine, z.B. Bradykinin, Kallidin, Histamin, und die NOS-Synthase, die aus L-Arginin das vasodilatatorisch wirkende NO freisetzt, zählen. Substanzen pflanzlichen Ursprungs wie der nachweislich vasodilatatorisch wirkenden Extrakt des Gingko biloba, DMSO, Xanthone, Flavonoide, Terpenoide, pflanzliche und tierische Farbstoffe, Lebensmittelfarben, NO-freisetzende Substanzen wie z.B. das Pentaerythrytiltetranitrat (PETN). Die oben erwähnten Kontrastmittel und Kontrastmittelanaloga zählen ebenfalls zu dieser Kategorie.

Die Löcher, Öffnungen und/oder Kanäle werden derart mit einem Wirkstoff oder einem Wirkstoff-enthaltenden Gemisch befüllt, dass sich der Inhalt relativ schnell löst und freigesetzt wird und somit nach Implantation des Stents die Löcher, Öffnungen und/oder Kanäle als erstes freigelegt bzw. geöffnet werden. Der sich in den Löchern, Öffnungen und/oder Kanälen befindende Wirkstoff wird sehr schnell abgegeben, so dass von einem Schnellrelease, d.h. einer

schnellen Freisetzung gesprochen werden kann, welche vorzugsweise wenige Stunden bis 2 Tage dauert.

Somit kann man das Problem der Restenose bzw. das gezielte Einwachsen des Stent in die Gefäßwand mittels anfänglicher Wirkstofffreisetzung steuern.

Diese schnelle Wirkstofffreisetzung lässt sich ferner mit einer langsamen Wirkstofffreisetzung kombinieren, wobei es sich um denselben oder einen anderen Wirkstoff handeln kann. Dieser Wirkstoff wird in die polymere Beschichtung eingebracht, so dass die polymere Beschichtung ferner auch als Wirkstoffträger wirkt.

In der polymeren Beschichtung ist vorzugsweise eine cytostatische Dosis eines antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoffs enthalten. Dieser Wirkstoff wird dann langsam in dem Maße freigesetzt, wie die polymere Beschichtung biologisch abgebaut wird.

Somit ermöglicht der erfindungsgemäße bioresorbierbare Stent zusätzlich auch noch die Möglichkeit einer Wirkstofffreisetzung und insbesondere der Kombination einer schnellen mit einer langsamen Wirkstofffreisetzung. Zusätzlich kann durch die Einhüllung des Stentkörpers auf Lumenseite gezielt Wirkstoffe einsetzen, die der Plättchenadhäsion bzw. Thrombusbildung entgegenwirken. Derartige Möglichkeiten lassen eine gezielte an die Umgebung spezifisch angepasste Wirkstoffabgabe und ebenso Wirkstoffkombinationen zu. Die Wirkstoffe lassen sich auf demselben Stent zielorientiert und voneinander unabhängig einsetzen.

Somit bietet die erfindungsgemäße Gefäßstütze eine Reihe von entscheidenden Vorteilen gegenüber den bekannten Ausführungsformen. Zum einen verhindert die polymere Hülle ein Zerfallen und Auseinanderbrechen des metallischen Grundgerüsts, was schwerwiegende Folgen haben kann. Bei den besonders bevorzugten Ausführungsformen wird durch den schnelleren biologischen Abbau des inneren metallischen oder Metall-enthaltenden Gerüstes gegenüber der polymeren Beschichtung gewährleistet, dass sich zuerst das innere Gerüst auflöst und dessen Auflösungsprodukte kontrolliert freigesetzt und vom Gewebe resorbiert werden. Hat das Gefäß seine eigene Stützkraft wiedererlangt, ist das innere Gerüst bereits im Auflösungszustand. Nach Auflösung der inneren Struktur baut sich danach auch die polymere Außenhülle biologisch ab.

Aufgrund der Struktur der polymeren Außenhülle mit Löchern, Öffnungen, Kanälen und/oder Poren erhält man zudem ein System, welches gezielt eine schnelle und eine langsame Wirkstofffreisetzung desselben oder unterschiedlicher Wirkstoffe miteinander vereint.

Die Löcher, Öffnungen, Kanäle und/oder Poren können gezielt mit einem Wirkstoff oder einem Gemisch enthaltend einen Wirkstoff befüllt werden und der Wirkstoff kann schnell aus diesen Kavitäten freigesetzt werden oder die gesamte Oberfläche oder ein Teil der Oberfläche der äußeren Polymerhülle wird mit einem Wirkstoff oder einem Gemisch enthaltend einen Wirkstoff beschichtet. Hier sind beliebige Ausführungsformen denkbar und praktikabel.

Ferner besteht die Möglichkeit, in die polymere bioabbaubare Beschichtung einen oder auch mehrere weitere Wirkstoffe einzulagern, welche dann langsam in dem Maße freigesetzt werden, wie sich die polymere Außenhülle auflöst, d.h. biologisch abgebaut wird.

Dieses System ist sehr flexibel, biete die Vorteile eines herkömmlichen Drug-Eluting-Stents und kombiniert darüber hinaus noch eine Schnellbehandlung mit einem Wirkstoff mit einer lokalen Langzeittherapie und ist zudem vollständig biologisch degradierbar, so dass nach einer gewissen Zeit kein Fremdkörper mehr im Körper des Patienten vorhanden ist. Damit wird z.B. das aktuell die Fachwelt sehr beunruhigende Problem der "late stent thrombosis" zu 100% vermieden.

Die erfindungsgemäße resorbierbare Gefäßstütze kann beispielsweise zu mindestens 30 Gew.-%, bevorzugt mindestens 40 Gew.-%, weiter bevorzugt mindestens 50 Gew.-%, weiter bevorzugt mindestens 60 Gew.-%, noch weiter bevorzugt mindestens 70 Gew.-%, noch weiter bevorzugt mindestens 80 Gew.-% und insbesondere bevorzugt mindestens 90 Gew.-% aus dem Metall Zink, Calcium, Magnesium oder Eisen bestehen.

Ferner ist bevorzugt, wenn das erfindungsgemäße Implantat zudem 0 - 60 Gew.-%, bevorzugt 0,01 - 59 Gew.-%, weiter bevorzugt 0,1 - 59 Gew.-%, noch weiter bevorzugt 1 - 58 Gew.-% Calcium aufweist. Insbesondere bevorzugt liegt die Masse an Calcium im Bereich von 1,5 - 50 Gew.-%, 2,0 - 40 Gew.-%, 2,5 - 30 Gew.-%, 3,0 - 20 Gew.-% und insbesondere von 3,5 - 10 Gew.-%.

Anstelle von Calcium oder in Kombination mit Calcium kann das erfindungsgemäße Implantat 0 - 80 Gew.-%, bevorzugt 0,01 - 70 Gew.-%, weiter bevorzugt 0,1 - 60 Gew.-%, noch weiter bevorzugt 1 - 50 Gew.-% Magnesium enthalten. Vorzugsweise liegt die Masse an Magnesium im Bereich von 0,1 - 80 Gew.-%, 5,0 - 70 Gew.-%, 7,5 - 60 Gew.-%, 10,0 - 50 Gew.-% und insbesondere im Bereich von 20 - 40 Gew.-%.

Neben Zink und/oder Eisen und optional Calcium und/oder Magnesium kann eine erfindungsgemäße Gefäßstütze ferner mindestens ein Metall ausgewählt aus der Gruppe umfassend Lithium, Natrium, Magnesium, Aluminium, Kalium, Calcium, Scandium, Titan, Vanadium, Chrom, Mangan, Eisen, Cobalt, Nickel, Kupfer, Zink, Gallium, Silizium, Yttrium, Zirconium, Niobium, Molybdän, Technetium, Ruthenium, Rhodium, Palladium, Silber, Indium, Zinn, Lanthan, Cer, Praseodym, Neodym, Promethium, Samarium, Europium, Gadolinium, Terbium, Dysprosium, Holium, Erbium, Thulium, Ytterbium, Lutetium, Tantal, Wolfram, Rhenium, Platin, Gold, Blei und/oder mindestens ein Metallsalz mit einem Kation ausgewählt aus der Gruppe umfassend $Li^+$, $Na^+$, $Mg^{2+}$, $K^+$, $Ca^{2+}$, $Sc^{3+}$, $Ti^{2+}$, $Ti^{4+}$, $V^{2+}$, $V^{3+}$, $V^{4+}$, $V^{5+}$,

$Cr^{2+}$, $Cr^{3+}$, $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Co^{2+}$, $Co^{3+}$, $Ni^{2+}$, $Cu^{+}$, $Cu^{2+}$, $Zn^{2+}$, $Ga^{+}$, $Ga^{3+}$, $Al^{3+}$, $Si^{4+}$, $Y^{3+}$, $Zr^{2+}$, $Zr^{4+}$, $Nb^{2+}$, $Nb^{4+}$, $Nb^{5+}$, $Mo^{4+}$, $Mo^{6+}$, $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$, $Ru^{7+}$, $Ru^{8+}$, $Rh^{3+}$, $Rh^{4+}$, $Pd^{2+}$, $Pd^{3+}$, $Ag^{+}$, $In^{+}$, $In^{3+}$, $Ta^{4+}$, $Ta^{5+}$, $W^{4+}$, $W^{6+}$, $pt^{2+}$, $Pt^{3+}$, $Pt^{4+}$, $Pt^{5+}$, $pt^{6+}$, $Au^{+}$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$, $Yb^{3+}$ enthalten. Neben den vorgenannten Metallen und Metallsalzen, welche zusammen in Massen von weniger als 5 Gew.-% anwesend sind, können geringe Mengen an Nichtmetallen, Kohlenstoff, Schwefel, Stickstoff, Sauerstoff und/oder Wasserstoff zugegen sein.

Insbesondere die Anwesenheit von Yttrium in Mengen von 0,01 - 10 Gew.-%, bevorzugt 0,1 - 9 Gew.-%, weiter bevorzugt 0,5 - 8 Gew.-%, weiter bevorzugt 1,0 - 7,0 Gew.-%, weiter bevorzugt 2,0 - 6,0 Gew.-% und insbesondere bevorzugt 3,0 - 5,0 Gew.-% kann vorteilhaft sein.

Eine bevorzugte Zusammensetzung eines erfindungsgemäßen Implantats umfasst beispielsweise

| | |
|---|---|
| 50 Gew.-% - 100 Gew.-% | Zink |
| 0,0 Gew.-% - 50 Gew.-% | Magnesium |
| 0,0 Gew.-% - 50 Gew.-% | Calcium |
| 0,0 Gew.-% - 10 Gew.-% | Yttrium |
| 0,0 Gew.-% - 10 Gew.-% | Seltene Erden |
| 0,0 Gew.-% - 5 Gew.-% | andere Metalle, Metallsalze, Nichtmetalle, Kohlenstoff, Schwefel, Stickstoff, Sauerstoff, Wasserstoff. |

Der Kohlenstoff, Schwefel, Stickstoff, Sauerstoff, Wasserstoff oder andere Nichtmetalle oder Halbmetalle können in Form von Anionen und/oder Polymeren vorliegen.

Weitere bevorzugte Zusammensetzungen sind:

| | |
|---|---|
| 55 Gew.-% - 100 Gew.-% | Zink |
| 0,1 Gew.-% - 40 Gew.-% | Magnesium |
| 0,1 Gew.-% - 40 Gew.-% | Calcium |
| 0,01 Gew.-% - 9 Gew.-% | Yttrium |
| 0,01 Gew.-% - 7 Gew.-% | Seltene Erden |
| 0,01 Gew.-% - 4 Gew.-% | andere Metalle, Metallsalze, Nichtmetalle, Kohlenstoff, Schwefel, Stickstoff, Sauerstoff, Wasserstoff. |

| | |
|---|---|
| 75 Gew.-% - 95 Gew.-% | Zink |
| 0,01 Gew.-% - 15 Gew.-% | Magnesium |
| 0,01 Gew.-% - 15 Gew.-% | Calcium |
| 0,01 Gew.-% - 6 Gew.-% | Yttrium |
| 0,01 Gew.-% - 3 Gew.-% | Seltene Erden |
| 0,01 Gew.-% - 2 Gew.-% | andere Metalle, Metallsalze, Nichtmetalle, Kohlenstoff, Schwefel, Stickstoff, Sauerstoff, Wasserstoff. |

| | |
|---|---|
| 41 Gew.-% - 91 Gew.-% | Zink |
| 7,0 Gew.-% - 55 Gew.-% | Magnesium |
| 0,00 Gew.-% - 10 Gew.-% | Calcium |
| 0,00 Gew.-% - 6 Gew.-% | Yttrium |
| 0,01 Gew.-% - 2 Gew.-% | Seltene Erden, andere Metalle, Metallsalze, Nichtmetalle, Kohlenstoff, Schwefel, Stickstoff, Sauerstoff, Wasserstoff. |

| | |
|---|---|
| 30 Gew.-% - 93 Gew.-% | Zink |
| 0,00 Gew.-% - 10 Gew.-% | Magnesium |
| 2,0 Gew.-% - 69 Gew.-% | Calcium |
| 0,00 Gew.-% - 6 Gew.-% | Yttrium |
| 0,01 Gew.-% - 2 Gew.-% | Seltene Erden, andere Metalle, Metallsalze, Nichtmetalle, Kohlenstoff, Schwefel, Stickstoff, Sauerstoff, Wasserstoff. |

(fortgesetzt)

| | |
|---|---|
| 55 Gew.-% - 100 Gew.-% | Eisen |
| 0,1 Gew.-% - 40 Gew.-% | Magnesium |
| 0,1 Gew.-% - 40 Gew.-% | Calcium |
| 0,01 Gew.-% - 9 Gew.-% | Yttrium |
| 0,01 Gew.-% - 7 Gew.-% | Seltene Erden |
| 0,01 Gew.-% - 4 Gew.-% | andere Metalle, Metallsalze, Nichtmetalle, Kohlenstoff, Schwefel, Stickstoff, Sauerstoff, Wasserstoff. |

| | |
|---|---|
| 55 Gew.-% - 100 Gew.-% | Eisen |
| 0,1 Gew.-% - 40 Gew.-% | Zink |
| 0,1 Gew.-% - 40 Gew.-% | Calcium |
| 0,01 Gew.-% - 9 Gew.-% | Yttrium |
| 0,01 Gew.-% - 7 Gew.-% | Seltene Erden |
| 0,01 Gew.-% - 4 Gew.-% | andere Metalle, Metallsalze, Nichtmetalle, Kohlenstoff, Schwefel, Stickstoff, Sauerstoff, Wasserstoff. |

| | |
|---|---|
| 55 Gew.-% - 100 Gew.-% | Eisen |
| 0,1 Gew.-% - 40 Gew.-% | Zink |
| 0,1 Gew.-% - 40 Gew.-% | Magnesium |
| 0,01 Gew.-% - 9 Gew.-% | Yttrium |
| 0,01 Gew.-% - 7 Gew.-% | Seltene Erden |
| 0,01 Gew.-% - 4 Gew.-% | andere Metalle, Metallsalze, Nichtmetalle, Kohlenstoff, Schwefel, Stickstoff, Sauerstoff, Wasserstoff. |

| | |
|---|---|
| 0,1 Gew.-% - 30 Gew.-% | Eisen |
| 0,1 Gew.-% - 30 Gew.-% | Zink |
| 0,1 Gew.-% - 30 Gew.-% | Calcium |
| 0,1 Gew.-% - 30 Gew.-% | Magnesium |
| 0,01 Gew.-% - 10 Gew.-% | Yttrium |
| 0,01 Gew.-% - 4 Gew.-% | Seltene Erden |
| 0,01 Gew.-% - 4 Gew.-% | andere Metalle, Metallsalze, Nichtmetalle, Kohlenstoff, Schwefel, Stickstoff, Sauerstoff, Wasserstoff. |

| | |
|---|---|
| 55,0 Gew.-% - 75,0 Gew.-% | Magnesium |
| 10,0 Gew.-% - 20,0 Gew.-% | Calcium |
| 5,0 Gew.-% - 15,0 Gew.-% | Yttrium |
| 5,0 Gew.-% - 10,0 Gew.-% | andere Metalle oder Metallsalze oder Seltene Erden |
| 0,5 Gew.-% - 10,0 Gew.-% | Nichtmetalle, Kohlenstoff, Schwefel, Stickstoff, Sauerstoff, Wasserstoff. |

| | |
|---|---|
| 20,0 Gew.-% - 40,0 Gew.-% | Magnesium |
| 20,0 Gew.-% - 40,0 Gew.-% | Calcium |
| 20,0 Gew.-% - 40,0 Gew.-% | Zink |
| 0,0 Gew.-% - 5,0 Gew.-% | Yttrium |
| 0,1 Gew.-% - 5,0 Gew.-% | andere Metalle oder Metallsalze oder Seltene Erden |
| 0,1 Gew.-% - 5,0 Gew.-% | Nichtmetalle, Kohlenstoff, Schwefel, Stickstoff, Sauerstoff, Wasserstoff. |

| | |
|---|---|
| 80 Gew.-% - 95 Gew.-% | Magnesium |
| 0,0 Gew.-% - 4 Gew.-% | Calcium |
| 0,1 Gew.-% - 4 Gew.-% | Yttrium |
| 0,0 Gew.-% - 4 Gew.-% | andere Metalle oder Metallsalze oder Seltene Erden |
| 0,1 Gew.-% - 4 Gew.-% | Nichtmetalle, Kohlenstoff, Schwefel, Stickstoff, Sauerstoff, Wasserstoff. |

Bei den aufgeführten Zusammensetzungen versteht sich von selbst, dass die Summe aller Bestandteile insgesamt 100,00 Gew.-% ergeben muss. Darüber hinaus können diese Zusammensetzungen noch einen sehr geringen Anteil an teilweise nicht vermeidbaren Verunreinigungen enthalten.

Als andere Metalle werden vorzugsweise unter anderem Titan, Zirkon, Niob, Tantal, Silizium, Lithium, Natrium, Kalium und Mangan und als Nichtmetalle vorzugsweise Kohlenstoff, Stickstoff und Sauerstoff bezeichnet.

Der Begriff "resorbierbar" bei der vorliegenden Erfindung bedeutet, dass das Implantat sich über eine gewisse Zeit im Organismus langsam auflöst und irgendwann nur noch dessen Abbauprodukte im Körper in gelöster Form vorliegen. Zu diesem Zeitpunkt sind feste Bestandteile oder Fragmente des Implantats nicht mehr vorhanden. Die Abbauprodukte sollten physiologisch weitgehend unbedenklich sein und zu Ionen oder Molekülen führen, welche im Organismus sowieso vorhanden oder vom Organismus zu unbedenklichen Stoffen abgebaut oder ausgeschieden werden können.

Als Metalle, welche in Kombination mit dem Zink eingesetzt werden können, sind erfindungsgemäß die folgenden bevorzugt: Lithium, Natrium, Magnesium, Aluminium, Kalium, Calcium, Scandium, Titan, Vanadium, Chrom, Mangan, Eisen, Cobalt, Nickel, Kupfer, Zink, Gallium, Silizium, Yttrium, Zirconium, Niobium, Molybdän, Technetium, Ruthenium, Rhodium, Palladium, Silber, Indium, Zinn, Lanthan, Cer, Praseodym, Neodym, Promethium, Samarium, Europium, Gadolinium, Terbium, Dysprosium, Holium, Erbium, Thulium, Ytterbium, Lutetium, Tantal, Wolfram, Rhenium, Platin, Gold, Blei. Insbesondere bevorzugt sind Magnesium, Calcium, Eisen, Yttrium. Ferner sind Kombinationen von Zink mit oder ohne den Zusatz eines oder mehrerer der vorgenannten Metalle mit Metallsalzen bevorzugt. Solche Kombinationen können als Metallsalz-enthaltende Zinkschmelzen oder Metallsalz-enthaltende Zinklegierungen bezeichnet werden. Der Metallsalzanteil darf nur so hoch sein, dass weiterhin eine ausreichende Flexibilität des Materials gegeben ist. Die Expandierbarkeit darf ebenfalls nicht wesentlich beeinträchtigt werden. Als Metallsalz eignen sich die weiter unten genannten und insbesondere Salze von Magnesium, Calcium, Eisen und Yttrium.

Besser als die Verwendung von Metallen ist hingegen die Verwendung von resorbierbaren Legierungen, welche beispielsweise folgende Metalle zusammen mit Zink enthalten können: Lithium, Natrium, Magnesium, Aluminium, Kalium, Calcium, Scandium, Titan, Vanadium, Chrom, Mangan, Eisen, Cobalt, Nickel, Kupfer, Zink, Gallium, Silizium, Yttrium, Zirconium, Niobium, Molybdän, Technetium, Ruthenium, Rhodium, Palladium, Silber, Indium, Zinn, Lanthan, Cer, Praseodym, Neodym, Promethium, Samarium, Europium, Gadolinium, Terbium, Dysprosium, Holium, Erbium, Thulium, Ytterbium, Lutetium, Tantal, Wolfram, Rhenium, Platin, Gold, Blei. Solche Metalle sind teilweise nur in geringen Mengen enthalten.

Bevorzugt sind Magnesium-Zink-Legierungen, welche Magnesium zu 10 bis 78 Gew.-%, bevorzugt 25 bis 68 Gew.-% und insbesondere bevorzugt über 36 bis 53 Gew.-% enthalten. Ferner ist bevorzugt, wenn diese Magnesium-Zink-Legierung des weiteren Scandium, Titan, Vanadium, Yttrium, Zirconium, Niobium, Molybdän, Technetium, Ruthenium, Rhodium, Palladium, Silber oder Indium und insbesondere Yttrium in einer Menge von 0,3 - 11, bevorzugt 0,7 - 10, weiter bevorzugt 1,1 - 8,5 und insbesondere bevorzugt 2 - 7 Gew.-% enthält.

Bevorzugt sind ferner Legierungen welche neben Zink zum überwiegenden Anteil Calcium, Magnesium, Eisen, Zinn, Zink oder Lithium zusammen mit bis zu 10 Gew.-% Scandium, Yttrium, Lanthan, Cer, Praseodym, Neodym, Promethium, Samarium, Europium, Gadolinium, Terbium, Dysprosium, Holmium, Erbium, Thulium und/oder Ytterbium enthalten.

Ferner sind insbesondere Metallsalz der oben genannten Metalle bevorzugt. Derartige Metallsalze enthalten vorzugsweise mindestens eines der folgenden Metallionen: $Li^+$, $Be^{2+}$, $Na^+$, $Mg^{2+}$, $K^+$, $Ca^{2+}$, $Sc^{3+}$, $Ti^{2+}$, $Ti^{4+}$, $V^{2+}$, $V^{3+}$, $V^{4+}$, $V^{5+}$, $Cr^{2+}$, $Cr^{3+}$ $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Co^{2+}$, $Co^{3+}$, $Ni^{2+}$, $Cu^+$, $Cu^{2+}$, $Zn^{2+}$, $Ga^+$, $Ga^{3+}$, $Al^{3+}$, $Si^{4+}$, $Y^{3+}$, $Zr^{2+}$, $Zr^{4+}$, $Nb^{2+}$, $Nb^{4+}$, $Nb^{5+}$, $Mo^{4+}$, $Mo^{6+}$ , $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$ , $Ru^{7+}$, $Ru^{8+}$, $Rh^{3+}$, $Rh^{4+}$, $Pd^{2+}$, $Pd^{3+}$, $Ag^+$, $In^+$, $In^{3+}$, $Ta^{4+}$, $Ta^{5+}$, $W^{4+}$, $W^{6+}$, $Pt^{2+}$, $Pt^{3+}$, $Pt^{4+}$, $Pt^{5+}$, $pt^{6+}$ , $Au^+$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$, $Yb^{3+}$.

Als Anionen dienen Halogene wie $F^-$, $Cl^-$, $Br^-$, Oxide und Hydroxide wie $OH^-$, $O^{2-}$, Sulfate, Carbonate, Oxalate, Phosphate wie $HSO_4^-$, $SO_4^{2-}$, $HCO_3^-$, $CO_3^{2-}$, $HC_2O_4^-$, $C_2O_4^{2-}$, $H_2PO_4^-$, $HPO_4^{2-}$, $PO_4^{3-}$, und insbesondere Carboxylate wie $HCOO^-$, $CH_3COO^-$, $C_2H_5COO^-$, $C_3H_7COO^-$, $C_4H_9COO^-$, $C_5H_{11}COO^-$, $C_6H_{13}COO^-$, $C_7H_{15}COO^-$, $C_8H_{17}COO^-$, $C_9H_{19}COO^-$, $PhCOO^-$, $PhCH_2COO^-$.

Des weiteren sind Salze der folgenden Säuren bevorzugt: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Äpfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure, Chininsäure, o-Methyl-mandelsäure, Hydrogenbenzolsulfonsäure, Methionin, Tryptophan, Lysin, Arginin, Pikrinsäure (2,4,6-Trinitrophenol), Adipinsäure, d-o-Tolylweinsäure,

Glutarsäure,

Ferner sind Salze von Aminosäuren bevorzugt, welche beispielsweise eine oder mehrere der folgenden Aminosäuren enthalten: Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N,N,N-Trimethyllysin, 3-Methylhistidin, 5-Hydroxylysin, O-Phosphoserin, γ-Carboxyglutamat, ε-N-Acetyllysin, ω-N-Methylarginin, Citrullin, Ornithin. Normalerweise werden die Aminosäuren mit L-Konfiguration eingesetzt. In einer weiteren bevorzugten Ausführungsform hat mindestens ein Teil der eingesetzten Aminosäuren D-Konfiguration.

Weitere bevorzugte resorbierbare Stoffe zur Herstellung des Implantats sind Metallsalze wie beispielsweise Caclicumchlorid, Calciumsulfat, Calciumphosphat, Calciumcitrat, Zinkchlorid, Zinksulfat, Zinkoxid, Zinkcitrat, Eisensulfat, Eisenphosphat, Eisenchlorid, Eisenoxid, Zink, Magnesiumchlorid, Magnesiumsulfat, Magnesiumphosphat oder Magnesiumcitrat. Derartige Metallsalze werden vorzugsweise in Massen von 0,01 - 12 Gew.-% eingesetzt.

Eine weitere bevorzugte Ausführungsform ist die Kombination von resorbierbarem Metall oder resorbierbarem Salz oder einer resorbierbaren Metalllegierung zusammen mit einem resorbierbaren Polymer. Eine solche Kombination kann bedeuten, dass das Implantat aus einer Mischung enthaltend Metall, Metalllegierung und/oder Metallsalz und einem biologisch abbaubaren Polymer hergestellt worden ist, oder das Implantat aus unterschiedlichen Schichten aufgebaut ist, wobei eine Schicht überwiegend oder ausschließlich das Metall, Metallsalz und/oder die Metalllegierung enthält und eine oder mehrere andere Schichten aus einem oder verschiedenen bioresorbierbaren Polymeren bestehen.

Die folgenden biologisch abbaubaren Polymere eignen sich insbesondere für die Herstellung der bioresorbierbaren Außenhülle. Diese resorbierbaren Polymere können aber auch dem Metall, Metallsalz oder der Metalllegierung beigesetzt werden, welche die innere Struktur bildet, wobei der Gewichtsanteil an organischen Polymeren 50 Gew.-% der gesamten inneren Struktur nicht überscheiten sollte und vorzugsweise bei weniger als 40 Gew.-%, weiter bevorzugt bei weniger als 30 Gew.-% und insbesondere bevorzugt bei weniger als 20 Gew.-% liegen sollte.

Als resorbierbare oder biologisch abbaubare Polymere können erfindungsgemäß folgende eingesetzt werden:

> Polydioxanon, Polycaprolacton, Polygluconat, Polymilchsäure-Polyethylenoxid-Copolymer, modifizierte Cellulose, Poly(hydroxybutyrat), Polyaminosäuren, Polyphosphatester, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-b-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate, Chitosane und Copolymere und/oder Mischungen der vorgenannten Polymere.

Insbesondere bevorzugte biologisch abbaubare Polymere sind Polydioxanon, Polycaprolacton, Polygluconat, Polyamide, Polymilchsäure-Polyethylenoxid-Copolymer, Polysaccharide wie z.B. Hyaluronsäure, Chitosan, regenerierte Cellulose, modifizierte Cellulose, Hydroxypropylmethylcellulose, Collagen, Gelatine, Polyhydroxybutyrat (PHBT) und Copolymere von Polyhydroxybutyrat, Polyanhydride (PAN), Polyphosphoester, Polyester, Polyaminosäuren, Polyglycolsäure, Poly-ε-Caprolacton, Polyphosphatester, Polyorthoester, Poly(L-lactid) (PLLA), Poly(D,L-lactid) (PLA), Polyglycolid (PGA), Poly(L-lactid-co-D,L-lactid) (PLLA/PLA), Poly(L-lactid-co-glycolid) (PLLA/PGA), Poly(D,L-lactid-co-glycolid) (PLA/PGA), Poly(glycolid-co-trimethylencarbonat) (PGA/PTMC), Polyethylenoxid (PEO), Polydioxanon (PDS), Polypropylenfumarat, Poly(ethylglutamat-co-glutaminsäure), Poly(tert-butyloxy-carbonylmethylglutamat), Polycaprolacton (PCL), Polycaprolacton-co-butylacrylat, Poly(phosphazen), Poly(D,L-lactid-co-caprolacton) (PLA/PCL), Poly(glycolid-co-caprolacton) (PGA/PCL), Maleinsäureanhydrid und Copolymere davon, Poly(aminosäuren), Polydepsipeptide, Maleinsäureanhydrid-Copolymere, Polyphosphazene, Polyiminocarbonate, Poly[(97.5% dimethyltrimethylencarbonat)-co-(2.5% trimethylencarbonat)], Cyanoacrylat, Polyethylenoxid, sowie Copolymere und Mischungen der vorgenannten Polymere.

Weiterhin bevorzugt sind mehrfach ungesättigte Fettsäuren, die über Autopolymerisation vernetzen wie z.B. Eicosapentaensäure, Timnodonsäure, Docosahexaensäure, Arachidonsäure, Linolsäure, α-Linolensäure, γ-Linolensäure sowie Mischungen der vorgenannten Fettsäuren und insbesondere Mischungen der reinen ungesättigten Verbindungen. Öle wie Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran enthalten ebenfalls hohe Anteile mehrfach ungesättigter Fettsäuren und sind deshalb ebenfalls einsetzbar.

Als weitere Substanzen für die polymere Beschichtung sind die Omega-3- als auch die Omega-6-Fettsäuren bevorzugt sowie sämtliche Substanzen, welche mindestens einen Omega-3- und/oder Omega-6-Fettsäurerest tragen. Derartige Substanzen zeigen eine gute Befähigung zur Autopolymerisation.

Die Fähigkeit auszuhärten, d.h. die Fähigkeit zur Autopolymerisation, ist in der Zusammensetzung der auch als trocknende Öle bezeichneten Öle begründet und geht auf den hohen Gehalt an essentiellen Fettsäuren, genauer auf die Doppelbindungen der ungesättigten Fettsäuren zurück. An der Luft werden mit Hilfe des Sauerstoffs an den Doppelbindungsstellen der Fettsäuremoleküle Radikale gebildet, die die radikalische Polymerisation initiieren und propagieren, so dass die Fettsäuren unter Verlust der Doppelbindungen untereinander vernetzen.

Die Autopolymerisation wird auch als Selbstpolymerisation bezeichnet und kann beispielsweise durch Sauerstoff, insbesondere Luftsauerstoff oder andere Radikalbildner initiiert werden. Eine weitere Möglichkeit besteht in der Initiierung der Autopolymerisation durch elektromagnetische Strahlung, insbesondere Licht. Weitere bevorzugte resorbierbare Polymere sind Polymethylmethacrylate (PMMA), Polytetrafluoroethylene (PTFE), Polyurethane, Polyvinylchloride (PVC), Polydimethylsiloxane (PDMS), Polyester, Nylons und Polylactide und Polyglycolide.

Bevorzugt sind insbesondere Polyester, Polylactide sowie Copoymere aus Diolen und Estern bzw. Diolen und Lactiden zur Herstellung der äußeren polymeren Hülle. Als Diole werden beispielsweise Ethan-1,2-diol, Propan-1,3-diol oder Butan-1,4-diol eingesetzt.

Erfindungsgemäß finden insbesondere Polyester für die polymere Schicht Verwendung. Aus der Gruppe der Polyester sind wiederum solche Polymere bevorzugt, welche die folgende Wiederholungseinheit besitzen:

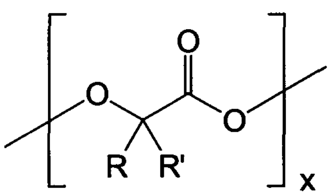

**Struktur A**

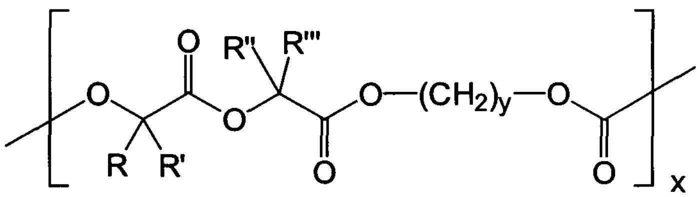

**Struktur B**

In den gezeigten Wiederholungseinheiten bedeutet R, R', R" und R"' einen Alkylrest mit 1 bis 5 Kohlenstoffatomen, insbesondere Methyl, Ethyl, Propyl, Isopropyl, n-Butyl, s-Butyl, t-Butyl, iso-Butyl, n-Pentyl oder Cyclopentyl und bevorzugt Methyl oder Ethyl. Y steht für eine ganze Zahl von 1 bis 9 und X steht für den Polymerisationsgrad. Insbesondere bevorzugt sind die folgenden Polymere mit den gezeigten Wiederholungseinheiten:

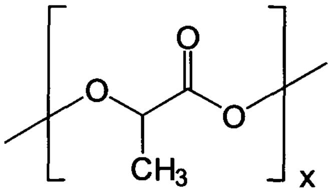

**Struktur A1**

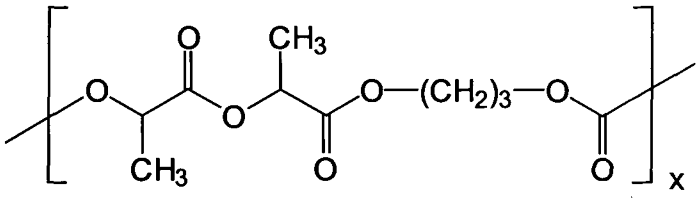

**Struktur B1**

Diese resorbierbaren Polymere werden auf der Basis von Milch- und Glycolsäure hergestellt. Grundsätzlich ist die Verwendung von resorbierbaren Polymer bei der vorliegenden Erfindung besonders bevorzugt. Homopolymere der Milchsäure (Polylactide) kommen hauptsächlich in der Produktion resorbierbarer, medizinischer Implantate zum Einsatz. Copolymere der Milch- und Glycolsäure finden als Rohstoffe für die Herstellung von Wirkstoffkapseln zur kontrollierten Freisetzung pharmazeutischer Wirksubstanzen Verwendung.

Somit sind für die erfindungsgemäße Verwendung insbesondere Polymere auf Milchsäure- und Glycolsäurebasis sowie Copolymere (alternierende oder statistische) und Blockcopolymere (z.B. Triblockcopolymere) beider Säuren bevorzugt.

Als weitere Vertreter der resorbierbaren Polymere seien als Resomere® bezeichneten bioresorbierbaren Polymere der Boehringer Ingelheim GmbH die Poly(L-lactid)e mit der allgemeinen Formel -(C6H8O4)n- wie L 210, L 210 S, L 207 S, L 209 S, die Poly(L-lactid-co-D,L-lactid)e mit der allgemeinen Formel -(C6H8O4)n-wie LR 706, LR 708, L 214 S, LR 704, die Poly(L-lactid-co-trimethylcarbonat)e mit der allgemeinen Formel -[(C6H804)x-(C4H603)y]n- wie LT 706, die Poly(L-lactid-co-glycolid)e mit der allgemeinen Formel -[(C6H804)x-(C4H404)y]n- wie LG 824, LG 857, die Poly(L-lactid-co-ε-caprolacton)e mit der allgemeinen Formel -[(C6H8O4)x-(C6H10O2)y]n- wie LC 703, die Poly(D,L-lactid-co-glycolid)e mit der allgemeinen Formel -[(C6H8O4)x-(C4H4O4)y]n- wie RG 509 S, RG 502 H, RG 503 H, RG 504 H, RG 502, RG 503, RG 504, die Poly(D,L-lactid)e mit der allgemeinen Formel -(C6H8O4)n- wie R 202 S, R 202 H, R 203 S und R 203 H. Resomer® 203 S stellt hierbei den Nachfolger des insbesondere bevorzugten Polymers Resomer® R 203 dar. Insbesondere bevorzugt ist die Verwendung von R203 und LT 706 in einem Mengenverhältnis von 70 Gew.-% zu 30 Gew.-%.

Es sei nochmals erwähnt, dass Gegenstand der vorliegenden Erfindung nicht die Bereitstellung eines bioresorbierbaren Stents oder die Bereitstellung einer bioresorbierbaren Mettalllegierung für einen Stent ist, sondern vielmehr die Kombination aus einem bioresorbierbaren Stentgerüst mit einer polymeren bioresorbierbaren Beschichtung, welche den Wassereintritt und den Ionenaustritt ermöglicht und bei den insbesondere bevorzugten Ausführungsformen das innere Stentgerüst sich deutlich schneller abbaut als die äußere Beschichtung.

Metalle und Metalllegierungen, welche zur Herstellung von biologisch abbaubaren Stentgerüsten geeignet sind, sind in der Literatur hinlänglich bekannt und grundsätzlich kann jede dieser Metalllegierungen, welche zumeist als Hauptbestandteil Magnesium, Zink, Calcium oder Eisen enthalten, verwendet werden.

Die Erfindung ist nun darin zu sehen, derartige biologisch abbaubare Metallgerüste mit einer polymeren Beschichtung zu versehen, welche die Abbauprodukte des inneren Stentgerüsts an die Umgebung abgeben, d.h. durch die polymere Beschichtung passieren lassen kann und in einer bevorzugten Ausführungsform sich selber erst dann beginnt aufzulösen, wenn das innere Stentgerüst bereist weitgehend biologisch abgebaut ist. Dies bedeutet, dass die polymere Beschichtung das innere Stentgerüst oder Fragmente des inneren Stentgerüsts während des biologischen Abbaus des inneren Stentgerüsts so lange sicher einschließt, bis der Stent in das umliegende Gewebe eingewachsen ist oder keine Herzinfarkt-auslösenden Fragmente des inneren Stentsgerüsts die polymere Beschichtung passieren können. Dieses Problem kann erfindungsgemäß durch eine Vielzahl von Ausführungsformen gelöst werden, wobei der Fachmann sowohl biologisch abbaubare Stentmaterialien bzw. Metalllegierungen als auch biologisch abbaubare polymere Beschichtungen kennt und diese nur gemäß der Lehre der vorliegenden Erfindung zu kombinieren sind. Sofern der Fachmann die Lehre gemäß vorliegender Erfindugn kennt, sind derartige Kombiantionen nicht mehr erfinderisch, sondern erfordern nur noch einige Standardtests, um die Ionendurchlässigkeit und die Abbaugeschwindigkeit von Stentgerüst und Polymerbeschichtung zu bestimmen.

Die Ionendurchlässigkeit lässt sich beispielsweise dadurch bestimmen, dass ein erfindungsgemäßer beschichteter Stent in einer wässrigen Lösung platziert wird und nach bestimmten Zeitabständen die elektrische Leitfähigkeit der Lösung gemessen wird, oder man den osmotischen Druck bestimmt oder mittels spektrokopischer Verfahren der Ionengehalt der Lösung bestimmt wird.

Eine insbesondere bevorzugte Ausführungsform der vorliegenden Erfindung ist auf Implantate mit einer inneren metallischen Struktur gerichtet, welche mit einem biologisch degradierbaren Polymer ausgewählt aus Polymethylmethacrylat (PMMA), Polytetrafluoroethylen (PTFE), Polyurethan, Polyvinylchlorid (PVC), Polydimethylsiloxan (PDMS), Polyester, Nylon oder Polylactid und insbesondere einem Polyester und/oder Polylactid beschichtet sind. Die polymere Beschichtung weist ferner bevorzugt Löcher, Öffnungen oder Kanäle auf, welche senkrecht zur Längsachse der jeweiligen Stentstrebe verlaufen.

Die Poren, Löcher, Öffnungen und/oder Kanäle sind vorzugsweise gleichmäßig über die Stentoberfläche verteilt und verlaufen weitgehend senkrecht durch das Polymer bis hin zum metallischen Innengerüst. Vorzugsweise sind 1 bis 20 derartiger Poren, Löcher, Öffnungen bzw. Kanäle pro $mm^2$ Stentstrebenoberfläche vorhanden.

Die gesamte Stentoberfläche, d.h. die Oberfläche der polymeren Hülle sowie der Poren, Löcher, Öffnungen und/oder Kanäle, oder ein Teil der Stentoberfläche und ein Teil der Poren, Löcher, Öffnungen und/oder Kanäle oder nur die Poren, Löcher, Öffnungen und/oder Kanäle oder auch nur ein Teil der Poren, Löcher, Öffnungen und/oder Kanäle können mit einem Wirkstoff oder einem Gemisch enthaltend mindestens einen Wirkstoff gefüllt werden.

Die polymere Beschichtung wird mittels bekannten Verfahren wie beispielsweise Sprühverfahren, Tauchverfahren, Plasmaverfahren, Pinselverfahren, Spritzenverfahren, Elektrospinning oder Pipettierverfahren auf die Struktur des Grundgerüstes aufgebracht und haftet daran auch vorzugsweise fest. Normalerweise werden nach erfolgter Beschichtung erst die Poren, Löcher, Öffnungen und/oder Kanäle in die Beschichtung mittels Laser, Temperatur, mechanischem Kontakt oder chemischem Einfluß eingebracht, wobei die Erzeugung der Poren, Löcher, Öffnungen und/oder Kanäle mittels Laser recht einfach ist aber nicht für alle Polymertypen das geeignetste Verfahren darstellt.

Weitere vorteilhafte Ausgestaltungen der vorliegenden Erfindung umfassen resorbierbare Implantate, welche in der biologisch abbaubaren Schicht und optional auf der biologisch abbaubaren Schicht mindestens eine pharmakologisch aktive Substanz enthalten. Als pharmakologisch aktive Substanzen sind antiproliferative, antimigrative, antiangiogene, antiinflammatorische, antiphlogistische, zytostatische, zytotoxische und/oder antithrombotische Wirkstoffe, Antirestenose-Wirkstoffe, Corticoide, Sexualhormone, Statine, Epothilone, Prostacycline, Angiogeneseinduktoren bevorzugt. Unter diesen Substanzen sind wiederum die antiproliferativen, antiinflammatorischen, antineoplastischen, antimigrativem, antiphlogistischen, zytostatischen, zytotoxischen und/oder antithrombotischen Wirkstoffe und die Antirestenose-Wirkstoffe bevorzugt.

Beispiele für antiinflammatorische, cytostatische, cytotoxische, antiproliferative, anti-mikrotubuli, antiangiogene, antirestenotische (anti-Restenose), antifungizide, antineoplastische, antimigrative, athrombogene und/oder antithrombogene Wirkstoffe sind: Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon $\alpha$-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, ß-Estradiol, $\alpha$-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate wie 6-$\alpha$-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, $\beta$-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor $X_a$-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon $\alpha$, ß und $\gamma$, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Fasudil, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

Bevorzugte Wirkstoffe sind Paclitaxel und dessen Derivate wie 6-$\alpha$-Hydroxy-Paclitaxel oder Baccatin oder andere Taxotere, Sirolimus, Everolimus, Biolimus A9, Pimecrolimus, Zotarolimus, Tacrolimus, Erythromycin, Midecamycin, Josamycin und Triazolopyrimidine.

Insbesondere bevorzugt sind Paclitaxel (Taxol®) sowie sämtliche Derivate von Paclitaxel wie beispielsweise 6-$\alpha$-Hydroxy-Paclitaxel und Sirolimus und seine Derivate.

Bei den erfindungsgemäßen resorbierbaren Implantaten handelt es sich bevorzugt um Stützprothesen für kanalartige Strukturen und insbesondere um Stents für Blutgefäße, Harnwege, Atemwege, Speiseröhre, Gallenwege oder den Verdauungstrakt.

Unter diesen Stents sind wiederum die Stents für Blutgefäße oder allgemeiner für das Herz- Kreislaufsystem bevorzugt.

In der Regel handelt es sich um selbstexpandierbare oder ballonexpandierbare Stents, welche vorzugsweise mindestens einen antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff vorzugsweise in der polymeren Beschichtung und/oder den Löchern, Öffnungen, Poren und/oder Kanälen enthalten.

Die bioabbaubare Schicht dienst in der Regel als Wirkstoffträger für den beispielsweise mindestens einen antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff. Dieser verhindert Entzündungen, welche durch den Stent verursacht werden können und reguliert das Wachstum von vor allem glatten Muskelzellen (koronare Endothelzellen) auf dem Stent. Der Stent ermöglicht eine Regeneration des gestützten Gewebes oder des gestützten Gefäßabschnittes. Hat sich das Gewebe regeneriert, kann es das Gefäß selbständig stützen und es bedarf keiner weiteren Unterstützung durch den Stent. Zu dieser Zeit ist der in die Gefäßwand eingewachsene Stent bereits deutlich abgebaut worden und in der Regel die innere Struktur bereits nicht mehr vorhanden. Die Abbauvorgänge setzten sich fort, bis der Stent sich vollständig aufgelöst hat, ohne jedoch dabei in feste Fragment zu zerfallen, welche sich in der Blutbahn frei bewegen könnten.

Die Begriffe "resorbierbar" oder "degradierbar" oder "bioabbaubar" oder "biologisch abbaubar" bezeichnen den Sachverhalt, dass der menschliche oder tierische Körper in der Lage ist, das Implantat langsam in Bestandteile aufzulösen, welche im Blut oder anderen Körperflüssigkeiten gelöst vorliegen.

Die bevorzugten Stents sind gitterartig ausgebildet, wobei die einzelnen Stege der Gitterstruktur ähnliche Querschnittsflächen aufweisen. Bevorzugt ist ein Verhältnis von größter zu kleinster Querschnittsfläche kleiner als 2. Die ähnlichen Querschnittsflächen der Stege führen dazu, dass der Stent gleichmäßig abgebaut wird.

Des weiteren ist bevorzugt, wenn die Stegringe durch Verbindungsstege verbunden sind, wobei die Verbindungsstege vorzugsweise eine kleinere Querschnittsfläche oder einen kleineren minimalen Durchmesser aufweisen als die Stege, die die Stegringe bilden. Dadurch wird erreicht, dass die Verbindungsstege im menschlichen oder tierischen Körper schneller abgebaut werden, als die Stegringe. Dadurch nimmt die axiale Flexibilität des Stents durch Abbau der Verbindungsstege schneller zu, als die Tragkraft des Stents in Folge des Abbaus der Stegringe abnimmt.

Das medizinische Implantat insbesondere der Stent kann mittels Sprüh-, Pipettier-, Pinsel-, Spritzen-, Plasmaabscheidungs-, Tauchverfahren, Elektrospinning oder "Seifenblasenverfahren" beschichtet werden, wobei ein Polymer in einem Lösungsmittel gelöst und diese Lösung auf das Implantat aufgetragen wird.

Das Polymer kann auch in Form eines Schlauches vorgeformt werden und auf der äusseren bzw. inneren Oberfläche des Stents aufgebracht werden.

Als Lösungsmittel für das bioresorbierbare Polymer eignen sich Wasser und bevorzugt organische Lösungsmittel wie beispielsweise Chloroform, Methylenchlorid (Dichlormethan), Aceton, Tetrahydrofuran (THF), Diethylether, Methanol, Ethanol, Propanol, Isopropanol, Diethylketon, Dimethylformamid (DMF), Dimethylacetamid, Essigsäuremethylester, Essigsäureethylester, Dimethylsulfoxid (DMSO), Benzol, Toluol, Xylol, t-Butylmethylether (MTBE), Petrolether (PE), Cyclohexan, Pentan, Hexan, Heptan, wobei Aceton, Chloroform und Essisäureethylester bevorzugt werden.

In einem geeigneten Lösungsmittel oder auch zusammen mit dem Polymer kann auch der mindestens eine aufzubringende Wirkstoff gelöst, emulgiert, suspendiert oder dispergiert werden. Als aufzubringende Substanzen kommen die oben erwähnten pharmakologisch aktiven Wirkstoffe sowie die oben beschriebenen Polymere in Frage.

Die polymere Beschichtung sollte relativ gleichmäßig erfolgen und eine Schichtdicke von 0,01 bis 10 μm besitzen. Die gewünschte Schichtdicke hängt auch vom jeweiligen Polymer ab und kann durch mehrmalige Beschichtungsschritte realisiert werden.

**Beispiele**

Beispiel 1 (nicht erfindungsgemäss):

Ermittlung des Elutionsverhaltens von Paclitaxel in PBS-Puffer

Je ein Stent wird in einem genügend kleinen Gefäß mit 2 ml PBS-Puffer versetzt, mit Parafilm verschlossen und im Trockenschrank bei 37°C inkubiert. Nach Ablauf der gewählten Zeitintervalle wird jeweils der Überstand abpipettiert und dessen UV-Absorption bei 306 nm gemessen.

Beispiel 2 (nicht erfindungsgemäss):

Biokompatible Beschichtung von biodegradierbaren Stents mit Leinöl und α-Linolensäure

Nach dem Reinigen der Stents mit Aceton und Ethanol wird eine in Ethanol gelöste Mischung mit 0,20% Leinöl und 0,5% α-Linolensäure hergestellt und gleichmäßig auf den Stent aufgesprüht. Der Stent wird bei einer Temperatur von

Beispiel 3 (nicht erfindungsgemäss):

Biokompatible Beschichtung von biodegradierbaren Stents mit Leinöl und dem synthetischen Polymeren Polyvinylpyrrolidon (PVP) im Zweischichtsystem unter Zugabe eines restenosehemmenden Wirkstoffes

Nach dem Reinigen der Stents wird eine erste Schicht aus 0,35Gew.-% Rapamycin gelöst in Chloroform auf den Stent aufgesprüht. Nach dem Trocknen dieser Schicht bei Raumtemperatur wird die zweite Schicht einer Chloroform-Lösung mit 0,25% Leinöl und 0,1% PVP aufgesprüht.

Beispiel 4 (nicht erfindungsgemäss):

Kovalente hämokompatible Beschichtung der biodegradierbaren Stents

**Beispiel 10a)**

Herstellung von desulfatiertem reacetyliertem Heparin:

100 ml Amberlite IR-122 Kationenaustauscherharz wurden in eine Säule mit 2 cm Durchmesser gefüllt, mit 400 ml 3M HCl in die $H^+$-Form überführt und mit destilliertem Wasser gespült, bis das Eluat chloridfrei und pH neutral war. 1 g Natrium-Heparin wurde in 10 ml Wasser gelöst, auf die Kationenaus-tauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH -Form und anschließend 150 μl Essigsäureanhydrid gegeben und 2 Stunden bei 4°C gerührt. Danach wird das Harz abfiltriert und die Lösung gegen Wasser dialysiert und gefriergetrocknet.

**Beispiel 10b)**

Kovalente Beschichtung der Stents

Die Stents wurden im Ultraschallbad 15 Minuten mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (v/v)) getaucht und anschließend für 5 Minuten bei 100°C getrocknet. Anschließend wurden die Stents über Nacht mit demineralisiertem Wasser gewaschen.

3 mg desulfatiertes und reacetyliertes Heparin wurden bei 4°C in 30 ml 0,1 M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung wurden 10 Stents für 15 Stunden bei 4°C gerührt. Anschließend wurde mit Wasser, 4M NaCl-Lösung und Wasser für je 2 Stunden gespült. Die Stents wurden ausgiebig im Luftstrom und im Vakkuumexsiccator getrocknet und gelagert.

Beispiel 5 (nicht erfindungsgemäss):

Beschichtung von biodegradierbaren Stents mit einem Wirkstoff in den Cavitäten der vollflächigen Matrix aus bioabbaubarem Polymer.

a)

- Beschichtung der Stents mit reiner Matrix mittels Sprühverfahren
  Herstellung der Sprühlösung :

  176 mg PLGA wird eingewogen und mit Chloroform auf 20 g aufgefüllt.

  Die Stents werden mit je 3 ml der Sprühlösung besprüht und anschliessend über Nacht getrocknet
  **oder**

- Beschichtung mit wirkstoffbeladener Matrix
  Sprühlösung : PLGA /'Taxol - Lösung wird aus 145,2 mg PLGA und 48,4 mg Taxol auf 22 g mit Chloroform aufgefüllt.
  Die Stents werden mit je 3 ml der Sprühlösung besprüht und anschliessend über Nacht getrocknet.

b) Erzeugung der Cavitäten im Pipettierverfahren

Nach dem vollständigen Trocknen der polymerbeschichteten Stents werden an der abluminalen Oberfläche des Stents durch punktuelles Anlösen der polymeren Schicht mit einer definierten Menge Chloroform oder einem anderen geeigneten Lösungsmittel Cavitäten in die Oberfläche derart erzeugt, dass diese sich gleichmässig über die Stentstreben des gesamten Stentkörpers verteilen. Eventuell noch vorhandene Lösungsmittelreste werden nach Erzeugung jeder Cavität im Luftstrom sofort entfernt.

c) Beschichtung der Cavitäten mit Wirkstoffbeladenem hydrophilen Polymeren im Pipettierverfahren

Mit Hilfe des Pipettierverfahrens werden die erzeugten Cavitäten mit einer viskosen wirkstoffbeladenen Lösung gefüllt. Die Lösung muss so viskos sein, dass sie nicht aus dem Hohlraum ausfliessen kann bzw. das Lösungsmittel derart schnell verdampft, dass sich die Lösung versteift als auch die umliegenden Matrix nicht aufgelöst wird.

Beispielsweise kann eine Rapamycin/PVP-Lösung verwendet werden, wobei der Anteil an Rapamycin in der Lösung 35% beträgt. In Kombination mit einem oder mehreren weiteren Wirkstoffen darf der Anteil von Rapamycin nicht unter 20% liegen. Der derart befüllte beschichtete Stent wird anschliessend getrocknet.

Beispiel 6 (nicht erfindungsgemäss):

Beschichtung der Cavitäten mit reinem Wirkstoff im Pipettierverfahren

Dazu werden 8,8 mg Taxol werden mit Chloroform auf 2 g aufgefüllt und in die Cavitäten pipettiert.

Beispiel 7 (nicht erfindungsgemäss):

Beschichtung der Cavitäten mit Wirkstoff und einem die Membrandurchgängigkeit beschleunigender Substanz im Pipettierverfahren

Dazu werden 450µl Ethanol mit 100µl Isopropylmyristat vermischt. Diese Lösung wird zu einer Lösung aus 4,5 ml Aceton und 150mg Epothilon A gegeben. Anschliessend werden mit Hilfe des Pipettierverfahrens die Cavitäten befüllt und getrocknet.

Beispiel 8 (nicht erfindungsgemäss):

Vollständige Beschichtung des zylindrischen Stentkörpers

a. Vorbeschichtung von Stents mit Sprühvorgang

Ein Stent entprechend Beispiel 1 bis 6 wird an den Stab eines Rotators befestigt und bei geringer Drehzahl mit sehr langsamen Auf- und Abbbewegungen der Pistole wird der Stent mit einer 1%igen Polyurethanlösung eingesprüht.

b. Vollflächige Beschichtung eines gesprühten Stents durch Tauchbeschichtung

Polyurethan wird in THF gelöst, so dass man eine 14 %ige Lösung erhält. Ein nach Beispiel 14b. vorbeschichteter Stent wird vorsichtig auf ein passendes Formwerkzeug aufgeschoben.

Das Werkzeug mit dem aufgezogenen Stent wird zunächst kurz in reines THF vorgetaucht. Danach taucht man langsam in die 14%ige Polyurethan-Lösung. Nach 15 Sekunden zieht man das Werkzeug mit dem Stent wieder langsam heraus und bringt ihn sofort in die Waagerechte und dreht weiter, damit sich das PU gleichmäßig auf dem Stent verteilt und trocknet.

Wenn es nicht mehr verläuft, wird der Kern unter dem Abzug trocknen gelassen und anschliessend bei 95°C im Trockenschrank getempert. Nach dem Abkühlen wird der Stent einschliesslich PU-Hülle sehr vorsichtig vom Werkzeug abgelöst. Dabei ist darauf zu achten, dass die PU-Hülle ohne Risse oder Löcher bleibt. Die Reinigung der derart vollflächig beschichteten Stents erfolgt sehr gründlich unter fließendem lauwarmen Wasser.

Beispiel 9 (nicht erfindungsgemäss):

Vollflächige Beschichtung eines gesprühten Stents mit PU/Tergurid durch Tauchbeschichtung

Die Tauchlösung besteht aus einer 30Gew-% Tergurid in Polymer, die dann mit THF auf 10% verdünnt wird. Die weitere Handhabung erfolgt wie in Beispiel 14b.

Beispiel 10 (nicht erfindungsgemäss):

Partielle Beschichtung von bioabbaubaren Stents (d=3mm)

Lösung: 3,2 mg PU gelöst in 20ml N-Methyl-2-Pyrrolidon

Ein sprühbeschichteter Stent wird auf ein passendes frei drehbares Formwerkzeug geschoben, so dass er vollständig auf dem glatten Untergrund aufliegt.

Das Auftragen der Beschichtung erfolgt in mindestens zwei Schichten, wobei mit einem Pinselhaar Lösung aufgenommen und diese in das zu beschichtende Feld aufgetragen wird, bis das Feld komplett mit Lösung bedeckt ist.

Wenn jedes ausgewählte zu beschichtende Feld in der gewünschten Beschichtungsdicke gefüllt ist, wird der Stent bei 90°C getrocknet. Nach dem Abkühlen wird der Stent vom Formwerkzeug gelöst.

Beispiel 11 (nicht erfindungsgemäss):

Ein bioresorbierbarer Stent folgender Zusammensetzung wird gemäß EP 1419793 B1 hergestellt:

| | |
|---|---|
| Magnesium | 91 Gew.-% |
| Yttrium | 4 Gew.-% |

(fortgesetzt)

| | |
|---|---|
| Neodym | 4 Gew.-% |
| sonstige | 1 Gew.-% |

"Sonstige" sind: andere Metalle, Metallsalze, Nichtmetalle, Kohlenstoff, Schwefel, Stickstoff, Sauerstoff, Wasserstoff.

Dieser Magnesiumstent wurde mit einer bioresorbierbaren Beschichtung aus PLLA/PGA versehen und die Abbaugeschwindigkeit des unbeschichteten Stents sowie des der polymeren Beschichtung aus PLLA/PGA auf einem Edelstahlstent als auch der beschichtete Stent gemäß Beispiel 17 wurden bestimmt.

Der unbeschichtete Magnesiumstent löste sich in PBS-Puffer (Phosphatpuffer mit 14,24 g $NaH_2PO_4$, 2,72 g $K_2HPO_4$ und 9 g NaCl; pH 7,4; T=37°C) innerhalb von 10 Tagen vollständig auf, während der beschichtete Stents gemäß Beispiel 17 sich im Inneren der Beschichtung innerhalb von ca. 12 Tagen vollständig auflöste.

Die PLLA/PGA-Beschichtung auf dem Edelstahlstent als auch auf dem Stent gemäß Beispiel 17 lösen sich im PBS-Puffer (Phosphatpuffer mit 14,24 g $NaH_2PO_4$, 2,72 g $K_2HPO_4$ und 9 g NaCl; pH 7,4; T=37°C) nach ca. 6 - 8 Wochen auf.

Eine optische Analyse eines Stents gemäß Beispiel 17 nach 10 Tagen, 20 Tagen, 30 Tagen und 40 Tagen im PBS-Puffer zeigt, dass nach 20 Tagen bereits das innere Stentgerüst vollständig aufgelöst und aus der Polymerbeschichtung herausgewaschen worden war, wobei der PLLA/PGA noch keine wesentlichen Auflösungserscheinungen und zumindest keine sichtbaren Löcher zeigte.

Beispiele 12:

Beschichtung der bioabbaubbaren Stents auf luminaler und abluminaler Seite mit zwei verschieden schnell abbaubaren Polylactiden (PLGA 75/25 und PLGA 50/50)

Der bioabbaubare Stent gemäss Beispiel 1 bis 6 wird horizontal auf eine dünne Metallstange (d= 0,2mm) gehängt, die auf die Rotationsachse der Rotations- und Vorschubanlage gesteckt ist, so dass die Innenseite des Stents die Stange nicht berührt. Während er langsam um seine Längsachse rotiert wird auf der abluminalen Oberfläche des Stents das schneller abbaubare Polymere (PLGA 50/50) gelöst in Chloroform auf die Stentstreben im kontinuierlichen Pipettierverfahren (wahlweise Pinselverfahren, Tintenstrahlverfahren, Kugelschreiberverfahren) aufgetragen. Die Trocknung erfolgt im leichten Luftstrom bei Raumtemperatur.

Der abluminal beschichtete Stent wird nun von der luminalen Seite mit dem langsamer abbaubaren Polylactid (PLGA 75/25) beschichtet. Hierzu werden die Stents entlang der Streben mit der Polymerlösung mittels eines Pinselhaars bestrichen. Anschliessend wird wieder bei Raumtemperatur im leichten Luftstrom getrocknet.

Polymerlösung : 176 mg Polylaktid wird eingewogen und mit Chloroform auf 20 g aufgefüllt.

Wahlweise lassen sich Wirkstoffe bzw. Wirkstoffkombinatonen in die Polymerlösung mischen. Beispielsweise ist ein entzündungshemmender, antiproliferativer Wirkstoff wie Rapamycin sehr gut für die abluminale, der Gefässwand zugewendete Seite sehr geeignet, während auf der luminalen, dem Blutstrom ausgesetzte Oberfläche ein antithrombogener Wirkstoff für die notwendige Thromboseprophylaxe sorgt, z.B.:

a) Abluminale Seite : Polylaktid PLGA 50/50 und Rapamycin (Lösung wird aus 145,2 mg Polylaktid und 48,4 mg Rapamycin auf 22 g mit Chloroform aufgefüllt).

b) Luminale Seite : Polylaktid PLGA 75/25 und Clopidogrel (Lösung wird aus 145,2 mg Polylaktid und 48,4 mg Clopidogrel auf 22 g mit Chloroform aufgefüllt).

Beispiel 13 (nicht erfindungsgemäss):

Biokompatible Beschichtung von bioabbaubaren Stents mit Leinöl und Paclitaxel

Leinöl und Paclitaxel (80:20) wird im Mischungsverhältnis 1:1 in Chloroform gelöst und dann auf den gleichmäßig rotierenden Stent gesprüht. Nach Abdampfen des Chloroform im leichten Luftstrom wird der Stent im Trockenschrank bei 80°C gelagert.

Beispiel 14 (nicht erfindungsgemäss):

Biokompatible Beschichtung von Stents mit Leinöl und Polyvinylpyrrolidon (PVP) im Zweischichtsystem unter Zugabe von Paclitaxel

Nach dem Reinigen der Stents wird eine erste Schicht aus 0,25Gew.% Paclitaxel gelöst in Chloroform auf den Stent aufgesprüht. Nach dem Trocknen dieser Schicht bei Raumtemperatur wird die zweite Schicht einer Chloroform-Lösung mit 0,25% Leinöl und 0,1% PVP aufgesprüht und über Nacht bei 70°C getrocknet.

Beispiel 15 (nicht erfindungsgemäss):

Messung der Degradation des bioresorbierbaren Stentmaterials

a) Herstellung der Legierung mittels Pulvermetallurgie

Die einzelnen Bestandteile werden verpulvert, gut durchmischt und mit hohem Druck in die gewünschte Form gepresst und anschliessend gesintert. Durch diese Herstellungsweise lassen sich unter Umgehung des Schmelzprozesses (Metallschmelze) kompakte und nahezu dichte Körper herstellen.

b) Degradationsmessung

Das als Röhrchen hergestellte und genau eingewogene (vier Stellen nach Komma) Werkstück wird ähnlich einem Stent in einem entsprechend geeigneten Silikonschlauch platziert. Die Schlauchenden befinden sich in einem mit PBS-Puffer (Phosphatpuffer mit 14,24 g $NaH_2PO_4$, 2,72 g $K_2HPO_4$ und 9 g NaCl; pH 7,4; T=37°C) gefüllten Behälter. Der Puffer wird über eine angeschlossene Schlauchpumpe aus dem Behälter durch den Schlauch gepumpt und wieder in den Behälter abgegeben, wobei am Schlauchende ein Filter dafür sorgt, dass eventuelle Partikel nicht im System umgewälzt werden können. Gleichzeitig dient der Filter als eine erste Kontrolle über die unerwünschte Entstehung und Ablösung grösserer Partikel während des Materialabbaus. Nach Ende der festgelegten Versuchszeit wird das Schlauchsegment mit dem verbliebenen Material vorsichtig herausgeschnitten, verlustfrei herausgeholt, getrocknet, gewogen und beschrieben.

Der zeitliche Verlauf des Abbaus einer Legierung wird anhand der Durchführung mehrerer der beschriebenen Versuchsaufbauten dokumentiert, die nach unterschiedlichen festgelegten Zeitintervallen beendet werden. Auf diese Weise lässt sich der Degradationsverlauf anhand des Gewichtsverlustes in Abhängigkeit von der Zeit erhalten (bei physiologischen T=37°C und pH 7,4 im nicht statischen System).

Beispiel 16 (nicht erfindungsgemäss):

Messung der Polymerdegradation im Fliesssystem

Der Abbau des bioabbaubaren Polymeren wird unter physiologischen Bedingungen (pH 7,4; T=37°C) in PBS-Puffer (Phosphatpuffer) untersucht. Hierzu wird das bioabbaubare Polymer zunächst in einem leichtflüchtigen Lösungsmittel wie Chloroform aufgelöst und anschliessend eine dünne Folie gegossen und bis zur Gewichtskonstanz im Vakuum getrocknet.

Die genau gewogene Folie wird in einer sogenannten Baumgartner-Kammer modifiziert nach Sakarassien [Sakariassen et al, J Lab- Clin. Med. 102(4) : 522 (1983)] (siehe Figur 2, Flachkammerperfusionssystem) eingebracht und PBS-Puffer wird mit einer ausgewählten Fliessgeschwindigkeit mit Hilfe einer Schlauchpumpe über die Folienoberfläche geleitet. Der Versuch wird mit unterschiedlichen Versuchszeiten durchgeführt und das Abbauverhalten anhand Foliengewicht (nach Trocknung unter Vakkuum bis zur Gewichtskonstanz) und Zustand bzw. Veränderungen der Polymercharakteristik festgehalten.

Beispiel 17 (nicht erfindungsgemäss):

Ein bioresorbierbarer Stent folgender Zusammensetzung wird gemäß EP 1419793 B1 hergestellt:

| | |
|---|---|
| Magnesium | 90 Gew.-% |
| Yttrium | 5 Gew.-% |
| Zirkonium | 4 Gew.-% |

(fortgesetzt)

| | |
|---|---|
| sonstige | 1 Gew.-% |

"Sonstige" sind: andere Metalle, Metallsalze, Nichtmetalle, Kohlenstoff, Schwefel, Stickstoff, Sauerstoff, Wasserstoff.

Dieser Magnesiumstent wurde mit einer bioresorbierbaren Beschichtung aus PGA/PTMC versehen und die Abbaugeschwindigkeit des unbeschichteten Stents sowie des der polymeren Beschichtung aus PGA/PTMC auf einem Edelstahlstent als auch der beschichtete Stent gemäß Beispiel 23 wurden bestimmt.

Der unbeschichtete Magnesiumstent löste sich in PBS-Puffer (Phosphatpuffer mit 14,24 g $NaH_2PO_4$, 2,72 g $K_2HPO_4$ und 9 g NaCl; pH 7,4; T=37°C) innerhalb von 13 Tagen vollständig auf, während der beschichtete Stents gemäß Beispiel 23 sich im Inneren der Beschichtung innerhalb von ca. 15 Tagen vollständig auflöste.

Die PGA/PTMC-Beschichtung auf dem Edelstahlstent als auch auf dem Stent gemäß Beispiel 23 lösen sich im PBS-Puffer (Phosphatpuffer mit 14,24 g $NaH_2PO_4$, 2,72 g $K_2HPO_4$ und 9 g NaCl; pH 7,4; T=37°C) nach ca. 15 - 18 Wochen auf.

Eine optische Analyse eines Stents gemäß Beispiel 23 nach 20 Tagen und 40 Tagen im PBS-Puffer zeigt, dass nach 20 Tagen bereits das innere Stentgerüst vollständig aufgelöst und aus der Polymerbeschichtung herausgewaschen worden war, wobei der PLLA/PGA noch keine wesentlichen Auflösungserscheinungen und zumindest keine sichtbaren Löcher zeigte. Die polymere Hülle war also noch vollständig in Takt, während sich das innere Stentgerüst vollständig aufgelöst hatte und die Metallionen duch das Polymere an die Pufferlösung abgegeben worden waren.

## Patentansprüche

1. Biologisch abbaubare Gefäßstütze aus einem inneren zumindest ein Metall enthaltenden, bioresorbierbaren Gerüst, welches von einer polymeren biologisch abbaubaren Beschichtung umgeben ist und wobei sich in der polymeren Beschichtung Mikroporen, Löcher, Öffnungen, Kanäle oder andere den Ionendurchtritt ermöglichende Strukturen befinden und wobei die luminale Beschichtung sich langsamer löst als die abluminale Stentbeschichtung.

2. Biologisch abbaubare Gefäßstütze nach Anspruch 1, wobei es sich bei dem inneren zumindest ein Metall enthaltenden, bioresorbierbaren Gerüst um ein Metall, Metalllegierung, Metalloxid, Metallsalz, Metallcarbid, Metallnitrid oder eine Mischung der vorgenannten Stoffe handelt.

3. Biologisch abbaubare Gefäßstütze nach Anspruch 1 oder 2, wobei das innere zumindest ein Metall enthaltende, bioresorbierbare Gerüst ein Potential von -0,53 eV gemessen im Vergleich zur Kalomelelektrode besitzt.

4. Biologisch abbaubare Gefäßstütze nach einem der vorherigen Ansprüche, wobei das innere zumindest ein Metall enthaltende, bioresorbierbare Gerüst schneller biologisch abbaubar ist als die polymere Außenhülle.

5. Biologisch abbaubare Gefäßstütze nach einem der vorherigen Ansprüche, wobei das innere zumindest ein Metall enthaltende, bioresorbierbare Gerüst mindestens ein Metall ausgewählt aus der Gruppe umfassend Lithium, Natrium, Magnesium, Aluminium, Kalium, Calcium, Scandium, Titan, Vanadium, Chrom, Mangan, Eisen, Cobalt, Nickel, Kupfer, Zink, Gallium, Silizium, Yttrium, Zirconium, Niobium, Molybdän, Technetium, Ruthenium, Rhodium, Palladium, Silber, Indium, Zinn, Lanthan, Cer, Praseodym, Neodym, Promethium, Samarium, Europium, Gadolinium, Terbium, Dysprosium, Holium, Erbium, Thulium, Ytterbium, Lutetium, Tantal, Wolfram, Rhenium, Platin, Gold, Blei enthält.

6. Biologisch abbaubare Gefäßstütze nach einem der vorherigen Ansprüche, wobei das innere zumindest ein Metall enthaltende, bioresorbierbare Gerüst mindestens ein Metallion der folgenden Oxidationsstufe umfasst: $Li^+$, $Na^+$, $Mg^{2+}$, $K^+$, $Ca^{2+}$, $Sc^{3+}$, $Ti^{2+}$, $Ti^{4+}$, $V^{2+}$, $V^{3+}$, $V^{4+}$, $V^{5+}$, $Cr^{2+}$, $Cr^{3+}$, $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Co^{2+}$, $Co^{3+}$, $Ni^{2+}$, $Cu^+$, $Cu^{2+}$, $Zn^{2+}$, $Ga^+$, $Ga^{3+}$, $Al^{3+}$, $Si^{4+}$, $Y^{3+}$, $Zr^{2+}$, $Zr^{4+}$, $Nb^{2+}$, $Nb^{4+}$, $Nb^{5+}$, $Mo^{4+}$, $Mo^{6+}$, $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$ , $Ru^{7+}$, $Ru^{8+}$, $Rh^{3+}$, $Rh^{4+}$, $Pd^{2+}$, $Pd^{3+}$, $Ag^+$, $In^+$, $In^{3+}$, $Ta^{4+}$, $Ta^{5+}$, $W^{4+}$, $W^{6+}$, $Pt^{2+}$, $Pt^{3+}$, $Pt^{4+}$, $Pt^{5+}$, $Pt^{6+}$, $Au^+$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$, $Yb^{3+}$.

7. Biologisch abbaubare Gefäßstütze nach Anspruch 5, wobei das Metall ausgewählt wird aus der Gruppe bestehend aus Magnesium, Calcium, Mangan, Eisen, Zink, Silizium, Yttrium, Zirconium und Gadolinium

8. Biologisch abbaubare Gefäßstütze nach einem der vorherigen Ansprüche, wobei die biologisch abbaubare Beschichtung aus mindestens einer der nachfolgend genannten bioabbaubaren Substanzen oder aus Mischungen der nachfolgend genannten bioabbaubaren Substanzen besteht: Polydioxanon, Polycaprolacton, Polygluconat, Polymilchsäure-Polyethylenoxid-Copolymer, modifizierte Cellulose, Poly(hydroxybutyrat), Polyaminosäuren, Polyphosphatester, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Polypara-dioxanone, Polyanhydride, Polymaleinsäureanhydride, Polyhydroxymethacrylate, Fibrin, Polycyanoacrylate, Polycaprolactondimethylacrylate, Poly-b-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(g-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Wachse, Öle, mehrfach ungesättigte Fettsäuren, die über Autopolymerisation vernetzten wie Eicosapentaensäure, Timnodonsäure, Docosahexaensäure, Arachidonsäure, Linolsäure, α-Linolensäure, γ-Linolensäure sowie Mischungen der vorgenannten Fettsäuren und Mischungen der reinen ungesättigten Verbindungen und Öle wie Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle bzw. Mischungen der reinen ungesättigten Verbindungen, Carrageenane, Fibrinogen, Agar-Agar, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate, Chitosane, und Copolymere und/oder Mischungen der vorgenannten Polymere.

**9.** Biologisch abbaubare Gefäßstütze nach Anspruch 8, wobei die biologisch abbaubare Beschichtung aus einem Polydioxanon, Polycaprolacton, Polygluconat, Polymilchsäure-Polyethylenoxid-Copolymer, modifizierte Cellulose, Collagen, Poly(hydroxybutyrat), Polyanhydrid, Polyphosphoester, Polyester, Polyaminosäuren, Polylactid, Polyglycolsäure, Poly-ε-Caprolacton und/oder Polyphosphatester besteht.

**10.** Biologisch abbaubare Gefäßstütze nach Anspruch 9, wobei die Mikroporen, Löcher, Öffnungen oder Kanäle mit einem antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff oder einer Zusammensetzung enthaltend einen antiinflammatorischen, cytostatischen, cytotoxischen, antiproliferativen, anti-mikrotubuli, antiangiogenen, antirestenotischen (anti-Restenose), antifungiziden, antineoplastischen, antimigrativen, athrombogenen und/oder antithrombogenen Wirkstoff gefüllt sind.

**11.** Biologisch abbaubare Gefäßstütze nach einem der vorherigen Ansprüche, wobei in der äußeren polymeren Beschichtung mindestens ein antiinflammatorischer, cytostatischer, cytotoxischer, antiproliferativer, anti-mikrotubuli, antiangiogener, antirestenotischer (anti-Restenose), antifungizider, antineoplastischer, antimigrativer, athrombogener und/oder antithrombogener Wirkstoff befindet.

**12.** Biologisch abbaubare Gefäßstütze nach Anspruch 10 oder 11, wobei der mindestens eine antiinflammatorische, cytostatische, cytotoxische, antiproliferative, anti-mikrotubuli, antiangiogene, antirestenotische (anti-Restenose), antifungizide, antineoplatische, antimigrative, athrombogene und/oder antithrombogene Wirkstoff aus der Gruppe ausgewählt wird, umfassend:

Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1 a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, b-myc-Antisense, ß-Lapachon,Podophyllotoxin, Podophyllsäure-2-ethylhydrazid, Molgramostim (rhuGM-CSF), Peginterferon $\alpha$-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, ß-Estradiol, $\alpha$-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate wie 6-$\alpha$-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, $\beta$-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Enoxoparin, desulfatiertes und N-reacetyliertes Heparin, Gewebe-Plasminogen-Aktivator, GpIIb/IIIa-Plättchenmembranrezeptor, Faktor $X_a$-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon $\alpha$, ß und$\gamma$ , Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin,

Fasudil Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

**13.** Biologisch abbaubare Gefäßstütze nach einem der vorherigen Ansprüche, wobei es sich bei der biologisch abbaubaren Gefäßstütze um einen Stent für Blutgefäße, Harnwege, Atemwege, Speiseröhre, Gallenwege oder den Verdauungstrakt handelt.

## Claims

**1.** Biodegradable vascular support consisting of an inner bioresorbable scaffold containing at least one metal and surrounded by a polymeric biodegradable coating, and wherein micropores, holes, openings, channels or other ion-permeable structures are included in the outer polymeric coating, and wherein the luminal coating dissolves slower than the abluminal stent coating.

**2.** Biodegradable vascular support according to claim 1, wherein the inner bioresorbable scaffold containing at least one metal is a metal, metal alloy, metal oxide, metal salt, metal carbide, metal nitride or a mixture of the aforementioned substances.

**3.** Biodegradable vascular support according to claim 1 or 2, wherein the inner bioresorbable scaffold containing at least one metal has a potential of -0.53 eV measured in comparison to the calomel electrode.

**4.** Biodegradable vascular support according to one of the previous claims, wherein the inner bioresorbable scaffold containing at least one metal is faster biodegradable than the polymeric outer wrapper.

**5.** Biodegradable vascular support according to one of the previous claims, wherein the inner bioresorbable scaffold containing at least one metal contains at least one metal selected from the group comprising lithium, sodium, magnesium, aluminum, potassium, calcium, scandium, titanium, vanadium, chromium, manganese, iron, cobalt, nickel, copper, zinc, gallium, silicon, yttrium, zirconium, niobium, molybdenum, technetium, ruthenium, rhodium, palladium, silver, indium, tin, lanthanum, cerium, praseodymium, neodymium, promethium, samarium, europium, gadolinium, terbium, dysprosium, holmium, erbium, thulium, ytterbium, lutetium, tantalum, tungsten, rhenium, platinum, gold, lead.

**6.** Biodegradable vascular support according to one of the previous claims, wherein the inner bioresorbable scaffold containing at least one metal comprises at least one metal ion of the following oxidation stage: $Li^{+}$, $Na^{+}$, $Mg^{2+}$, $K^{+}$, $Ca^{2+}$, $Sc^{3+}$, $Ti^{2+}$, $Ti^{4+}$, $V^{2+}$, $V^{3+}$, $V^{4+}$, $V^{5+}$, $Cr^{2+}$, $Cr^{3+}$, $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Co^{2+}$, $Co^{3+}$, $Ni^{2+}$, $Cu^{+}$, $Cu^{2+}$, $Zn^{2+}$, $Ga^{+}$, $Ga^{3+}$, $Al^{3+}$, $Si^{4+}$, $Y^{3+}$, $Zr^{2+}$, $Zr^{4+}$, $Nb^{2+}$, $Nb^{4+}$, $Nb^{5+}$, $Mo^{4+}$, $Mo^{6+}$ , $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$, $Ru^{7+}$, $Ru^{8+}$, $Rh^{3+}$, $Rh^{4+}$, $Pd^{2+}$, $Pd^{3+}$, $Ag^{+}$, $In^{+}$, $In^{3+}$, $Ta^{4+}$, $Ta^{5+}$, $W^{4+}$, $W^{6+}$, $Pt^{2+}$, $Pt^{3+}$, $Pt^{4+}$, $Pt^{5+}$, $Pt^{6+}$, $Au^{+}$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$, $Yb^{3+}$.

**7.** Biodegradable vascular support according to claim 5, wherein the metal is selected from the group consisting of magnesium, calcium, manganese, iron, zinc, silicon, yttrium, zirconium and gadolinium.

**8.** Biodegradable vascular support according to one of the previous claims, wherein the biodegradable coating consists of at least one of the following biodegradable substances or of mixtures of the following biodegradable substances: polydioxanone, polycaprolactone, polygluconate, poly(lactic acid) polyethylene oxide copolymer, modified cellulose, polyhydroxybutyrate, polyamino acids, polyphosphate ester, polyvalerolactones, poly-ε-decalactone, polylactonic acid, polyglycolic acid, polylactides, polyglycolides, copolymers of the polylactides and polyglycolides, poly_ε-caprolactone, polyhydroxybutyric acid, polyhydroxybutyrates, polyhydroxyvalerates, polyhydroxybutyrate-co-valerate, poly(1,4-dioxane-2,3-one), poly(1,3-dioxane-2-one), poly-para-dioxanone, polyanhydrides, polymaleic anhydrides, polyhydroxy methacrylates, fibrin, polycyanoacrylate, polycaprolactone dimethylacrylates, poly-ß-maleic acid, polycaprolactone butyl acrylates, multiblock polymers from oligocaprolactonediols and oligodioxanonediols, polyether ester multiblock polymers from PEG and poly(butylene terephthalates), polypivotolactones, polyglycolic acid trimethyl carbonates, polycaprolactone glycolides, poly(γ-ethyl glutamate), poly(DTH-iminocarbonate), poly(DTE-co-DT-carbonate), poly(bisphenol A-iminocarbonate), polyorthoesters, polyglycolic acid trimethyl carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes having amino acid residues in the backbone, polyetheresters such as polyethylene oxide, polyalkene oxalates, polyorthoesters as well as copolymers thereof, lipids, waxes, oils, polyunsaturated fatty acids crosslinked by auto-polymerisation such as eicosapentaenoic acid, timnodonic acid, docosahexaenoic acid, arachidonic acid, linoleic acid, α-linolenic acid, γ-linolenic acid as well as mixtures of the afore-mentioned fatty acids and mixtures of the pure unsaturated compounds and oils such as linseed oil, hemp oil, corn oil, walnut oil, rape oil, soy oil, sunflower oil, poppy-seed oil, safflower oil, wheat germ oil, thistle oil, grape seed oil, evening primrose oil, borage oil, black cumin oil, algae oil, fish oil, cod-liver oil and/or mixtures of the afore-mentioned oils or mixtures of the pure unsaturated compounds, respectively, carrageenans, fibrinogen, agar-agar, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivates thereof, heparins, chondroitin sulfate, dextran, ß-cyclodextrins, copolymers with PEG and polypropylene glycol, gum arabic, guar, gelatin, collagen N-hydroxysuccinimide, lipids, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylenes, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM gums, silicones such as polysiloxanes, polydimethylsiloxanes, polyvinyl halogens and copolymers, cellulose ethers, cellulose triacetates, chitosans and copolymers and/or mixtures of the aforementioned polymers.

**9.** Biodegradable vascular support according to claim 8, wherein the biodegradable coating consists of a polydioxanone, polycaprolactone, polygluconate, poly(lactic acid) polyethylene oxide copolymer, modified cellulose, collagen, polyhydroxybutyrate, polyanhydride, polyphosphoester, polyester, polyamino acids, polylactides, polyglycolides, poly_ε-caprolactone and/or polyphosphate ester.

**10.** Biodegradable vascular support according to claim 9, wherein the micropores, holes, openings or channels are filled with an anti-inflammatory, cytostatic, cytotoxic, antiproliferative, anti-microtubuli, antiangiogenic, antirestenotic (anti-restenosis), antifungicide, antineoplastic, antimigrative, athrombogenic and/or antithrombogenic agent or with a composition containing an anti-inflammatory, cytostatic, cytotoxic, antiproliferative, anti-microtubuli, antiangiogenic, antirestenotic (anti-restenosis), antifungicide, antineoplastic, antimigrative, athrombogenic and/or antithrombogenic agent.

**11.** Biodegradable vascular support according to one of the previous claims, wherein the outer polymeric coating contains at least one anti-inflammatory, cytostatic, cytotoxic, antiproliferative, anti-microtubuli, antiangiogenic, antirestenotic (anti-restenosis), antifungicide, antineoplastic, antimigrative, athrombogenic and/or antithrombogenic agent.

**12.** Biodegradable vascular support according to claim 10 or 11, wherein the at least one anti-inflammatory, cytostatic, cytotoxic, antiproliferative, anti-microtubuli, antiangiogenic, antirestenotic (anti-restenosis), antifungicide, antineoplastic, antimigrative, athrombogenic and/or antithrombogenic agent is selected from the group comprising:

abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics, antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, bombrestatin, Boswellic acids and derivatives thereof, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone,

daunorubicin, diclofenac, 1,11-dimethoxycanthin-6-one, docetaxel, doxorubicin, daunomycin, epirubicin, epothilone A and B, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclophosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, inhibitor 2ω of smc proliferation, mitoxanthrone, mycophenolate c-myc antisense, b-myc antisense, ß-lapachone, podophyllotoxin, podophyllic acid 2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, NFkB, angiopeptine, monoclonal antibodies inhibiting muscle cell proliferation, bFGF antagonists, probucol, prostaglandins, 1-hydroxy-11-methoxycanthin-6-one, scopoletin, NO donors such as pentaerythritol tetranitrate and sydnonimines, S-nitroso derivatives, tamoxifen, staurosporine, ß-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and derivatives thereof such as 6-α-hydroxy-paclitaxel, taxoteres, carbon suboxides (MCS) and macrocylic oligomers thereof, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, S 100 protein, bacitracin, vitronectin receptor antagonists, azelastine, guanidyl cyclase stimulator tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF 1, active agents from the group of antibiotics such as cefadroxil, cefazolin, cefaclor, cefoxitin, tobramycin, gentamicin, penicillins such as dicloxacillin, oxacillin, sulfonamides, metronidazole, enoxaparin, desulfated and N-reacetylated heparin, tissue plasminogen activator, GpIIb/IIIa platelet membrane receptor, antibodies to factor Xa inhibitor, heparin, hirudin, r-hirudin, PPACK, protamine, prourokinase, streptokinase, warfarin, urokinase, vasodilators such as dipyramidole, trapidil, nitroprussides, PDGF antagonists such as triazolopyrimidine and seramin, ACE inhibitors such as captopril, cilazapril, lisinopril, enalapril, losartan, thioprotease inhibitors, prostacyclin, vapiprost, interferon α, ß und γ, histamine antagonists, serotonin blockers, apoptosis inhibitors, apoptosis regulators such as p65, NF-kB or Bcl-xL antisense oligonucleotides, halofuginone, nifedipine, tocopherol, tranilast, molsidomine, tea polyphenols, epicatechin gallate, epigallocatechin gallate, leflunomide, etanercept, sulfasalazine, etoposide, dicloxacylline, tetracycline, triamcinolone, mutamycin, procainimide, retinoic acid, quinidine, disopyrimide, flecainide, propafenone, sotalol, natural and synthetically obtained steroids such as inotodiol, maquiroside A, ghalakinoside, mansonine, strebloside, hydrocortisone, betamethasone, dexamethasone, non-steroidal substances (NSAIDS) such as fenoprofen, ibuprofen, indomethacin, naproxen, phenylbutazone and other antiviral agents such as acyclovir, ganciclovir and zidovudine, clotrimazole, flucytosine, griseofulvin, ketoconazole, miconazole, nystatin, terbinafine, antiprotozoal agents such as chloroquine, mefloquine, quinine, moreover natural terpenoids such as hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceantinoside C, yadanziosides N and P, isodeoxyelephantopin, tomenphantopin A and B, coronarin A, B, C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, maquiroside A, marchantin A, maytansin, lycoridicin, margetine, pancratistatin, liriodenine, bisparthenolidine, oxoushinsunine, periplocoside A, ursolic acid, deoxypsorospermin, psychorubin, ricin A, sanguinarine, manwu wheat acid, methylsorbifolin, chromones of spathelia, stizophyllin, mansonine, strebloside, dihydrousambaraensine, hydroxyusambarine, strychnopentamine, strychnophylline, usambarine, usambarensine, liriodenine, oxoushinsunine, daphnoretin, lariciresinol, methoxylariciresinol, syringaresinol, sirolimus (rapamycin), somatostatin, tacrolimus, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

**13.** Biodegradable vascular support according to one of the previous claims, wherein the biodegradable vascular support is a stent for blood vessels, the urinary tract, the airways, oesophagus, bile ducts or the intestinal tract.

**Revendications**

1. Un support vasculaire biodégradable ayant un échafaudage intérieur biorésorbable contenant au moins un métal, ledit échafaudage étant entouré par un revêtement polymérique biodégradable, et où dans le revêtement polymérique se trouvent des micropores, des trous, des ouvertures, des canaux ou d'autres structures perméables aux ions et où le revêtement luminale se dissout plus lentement que le revêtement abluminale du stent.

2. Le support vasculaire biodégradable selon la revendication 1, dans lequel l'échafaudage intérieur biorésorbable contenant au moins un métal est un métal, un alliage métallique, un oxyde métallique, un sel métallique, un carbure métallique, un nitrure métallique ou un mélange des substances mentionnées ci-dessus.

3. Le support vasculaire biodégradable selon la revendication 1 ou 2, dans lequel l'échafaudage intérieur biorésorbable contenant au moins un métal a un potentiel de -0,53 eV mesuré par rapport à l'électrode au calomel.

4. Le support vasculaire biodégradable selon une quelconque des revendications précédentes, dans lequel l'échafaudage intérieur biorésorbable contenant au moins un métal est plus facilement biodégradable que l'enveloppe polymérique extérieure.

5. Le support vasculaire biodégradable selon une quelconque des revendications précédentes, dans lequel l'au moins un métal de l'échafaudage intérieur biorésorbable contenant au moins un métal est sélectionné à partir du groupe comprenant lithium, sodium, magnésium, aluminium, potassium, calcium, scandium, titan, vanadium, chrome, manganèse, fer, cobalt, nickel, cuivre, zinc, gallium, silicium, yttrium, zirconium, niobium, molybdène, technétium, ruthénium, rhodium, palladium, argent, indium, étain, lanthan, cérium, praséodyme, néodyme, prométhium, samarium, europium, gadolinium, terbium, dysprosium, holmium, erbium, thulium, ytterbium, lutétium, tantale, tungstène, rhénium, platine, or, plombe.

6. Le support vasculaire biodégradable selon une quelconque des revendications précédentes, dans lequel l'échafaudage intérieur biorésorbable contenant au moins un métal comprend au moins un ion métallique de l'état d'oxydation suivant : $Li^{+}$, $Na^{+}$, $Mg^{2+}$, $K^{+}$, $Ca^{2+}$, $Sc^{3+}$, $Ti^{2+}$, $Ti^{4+}$, $V^{2+}$, $V^{3+}$, $V^{4+}$, $V^{5+}$, $Cr^{2+}$, $Cr^{3+}$, $Cr^{4+}$, $Cr^{6+}$, $Mn^{2+}$, $Mn^{3+}$, $Mn^{4+}$, $Mn^{5+}$, $Mn^{6+}$, $Mn^{7+}$, $Fe^{2+}$, $Fe^{3+}$, $Co^{2+}$, $Co^{3+}$, $Ni^{2+}$, $Cu^{+}$, $Cu^{2+}$, $Zn^{2+}$, $Ga^{+}$, $Ga^{3+}$, $Al^{3+}$, $Si^{4+}$, $Y^{3+}$, $Zr^{2+}$, $Zr^{4+}$, $Nb^{2+}$, $Nb^{4+}$, $Nb^{5+}$, $Mo^{4+}$, $Mo^{6+}$, $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$, $Ru^{7+}$, $Ru^{8+}$, $Rh^{3+}$, $Rh^{4+}$, $Pd^{2+}$, $Pd^{3+}$, $Ag^{+}$, $In^{+}$, $In^{3+}$, $Ta^{4+}$, $Ta^{5+}$, $W^{4+}$, $W^{6+}$, $Pt^{2+}$, $Pt^{3+}$, $Pt^{4+}$, $Pt^{5+}$, $Pt^{6+}$, $Au^{+}$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Tb^{3+}$, $Pr^{3+}$, $Pm^{3+}$, $Sm^{3+}$, $Eu^{2+}$, $Dy^{3+}$, $Ho^{3+}$, $Er^{3+}$, $Tm^{3+}$, $Yb^{3+}$.

7. Le support vasculaire biodégradable selon la revendication 5, dans lequel le métal est sélectionné à partir du groupe consistant en magnésium, calcium, manganèse, fer, zinc, silicium, yttrium, zirconium et gadolinium.

8. Le support vasculaire biodégradable selon une quelconque des revendications précédentes, dans lequel le revêtement biodégradable consiste en au moins une des biodégradables substances mentionnées ci-dessous ou des mélanges des substances biodégradables mentionnés ci-dessous : polydioxanone, polycaprolactone, polygluconate, copolymère acide polylactique/poly(éthylène oxyde), cellulose modifiée, polyhydroxybutyrate, poly(amino acides), esters de polyphosphate, polyvalérolactones, poly-ε-décalactone, polylactones, acide polyglycolique, polylactides, polyglycolides, copolymères polylactides/polyglycolides, poly-ε-caprolactone, polyhydroxybutyrates, polyhydroxyvalérates, polyhydroxybutyrate-co-valérates, poly(1,4-dioxane-2,3-diones), poly(1,3-dioxane-2-ones), poly-para-dioxanones, polyanhydrides, poly(anhydride maléique), poly-hydroxy-méthacrylates, fibrine, poly-cyanoacrylates, polycaprolactone diméthylacrylates, acide poly-β-maléique, polycaprolacton-butyl-acrylates, polymères à blocs multiples d'oligocaprolactondiols et d'oligodioxanondiols, polyéther-ester-polymères à blocs multiples de PEG et de polybutylène téréphthalate, polypivotolactones, acide polyglycolique triméthyl carbonates, polycaprolactone glycolides, poly(γ-éthyl-glutamate), poly(DTH-iminocarbonate), poly(DTE-co-DT-carbonate), poly(bisphénol A-iminocarbonate), polyorthoesters, acide polyglycolique triméthyl carbonates, polytriméthyl carbonates, polyiminocarbonates, poly(N-vinyl)-pyrrolidone, polyvinyl alcohols, polyester amides, polyesters glycolés, polyphosphoesters, polyphosphazènes, poly[p-carboxyphénoxy)propane], acide polyhydroxy-péntanoique, polyanhydrides, polyéthylène oxyde propylène oxyde, polyuréthanes mous, polyuréthanes ayant des résidus amino acide dans le squelette, polyétheresters comme polyéthylène oxyde, polyalkènoxalates, polyorthoesters ainsi que copolymères de ceux-ci, lipides, cires, huiles, acides gras poly-insaturés, connectés par auto-polymérisation comme acide eicosapentaénoïque, acide timnodonique, acide docosahexaénoïque, acide arachidonique, acide linoléique, acide α-linoléique, acide γ-linoléique, ainsi que des mélanges des huiles mentionnées ci-dessus et des mélanges des composés insaturés et huiles comme l'huile de lin, l'huile de chanvre, l'huile de de germe de mais, l'huile de noix, l'huile de

colza, l'huile de soja, l'huilde de tournesol, l'huile de pavot, l'huile de carthame, l'huile de germe de blé, huile de pépins de raisin, l'huile d'onagre, l'huile de bourrache, l'huile de cumin noir, l'huile d'algues, l'huile de poisson, l'huile de foie de morue et/ou des mélanges des huiles mentionnées ci-dessus, respectivement mélanges des composés pures insaturés, carraghénane, fibrinogène, agar-agar, starch, collagène, polymères à base de protéine, poly(amino acides), poly(amino acides) synthétiques, zéine, polyhydroxyalkanoates, acide pectique, acide actinique, carboxyméthyl sulfate, albumine, acide hyaluronique, chitosane et ses dérivés, héparansulfate et ses dérivés, héparines, chondroïtine sulfate, dextrane, β-cyclodextrines, copolymères avec PEG et polypropylène glycol, gomme arabique, guar, gélatine, collagène, collagène N-hydroxysuccinimide, lipides, phospholipides, acide polyacrylique, polyacrylates, polyméthyl-méthacrylate, polybutyl-méthacrylate, polyacrylamide, polyacrylonitriles, polyamides, polyétheramides, polyéthylène amine, polyimides, polycarbonates, polycarbouréthanes, polyvinylcétones, polyvinyle halogénures, polyvinylidène halogénures, éthers polyvinyliques, polyisobutylènes, aromatiques polyvinyliques, esters polyvinyliques, pyrrolidones polyvinyliques, polyoxyméthylènes, polytétraméthylène oxyde, polyéthylène, polypropylène, polytétrafluoroéthylène, polyuréthanes, polyéther uréthanes, silicone polyéther uréthanes, silicone polyuréthanes, silicone polycarbonate uréthanes, polyoléfine élastomères, polyisobutylènes, gommes EPDM, fluorosilicones, carboxyméthyl chitosans, polyarylétheréthercétones, polyétheréthercétones, polyéthylène téréphthalate, polyvalérates, carboxyméthylcellulose, cellulose, rayonne, rayonne triacétates, cellulose nitrates, cellulose acétates, hydroxyéthyl cellulose, cellulose butyrates, cellulose acétate butyrates, éthyle vinyle acétate copolymères, polysulfones, résines époxy, résines ABS, silicones comme polysiloxanes, polydiméthylsiloxanes, polyvinyl-halogénes et copolymères, cellulose éthers, cellulose triacétates, chitosane, et copolymères et/ou mélanges des polymères mentionnés ci-dessus.

**9.** Le support vasculaire biodégradable selon la revendication 8, dans lequel le revêtement biodégradable consiste en polydioxanone, polycaprolactone, polygluconate, copolymère acide polylactique/poly(éthylène oxyde), cellulose modifiée, collagène, polyhydroxybutyrate, polyanhydride, polyphosphoester, polyester, poly(amino acides), polylactide, acide polyglycolique, poly-ε-caprolactone et/ou esters de polyphosphate.

**10.** Le support vasculaire biodégradable selon la revendication 9, dans lequel les micropores, les trous, les ouvertures ou les canaux sont remplis avec un agent actif anti-inflammatoire, cytostatique, cytotoxique, anti-prolifératif, anti-microtubules, anti-angiogénique, anti-resténotique (anti-resténose), anti-fongicide, anti-néoplasique, antimigrative, athrombogénique et / ou antithrombogénique ou une composition comprenant un agent actif anti-inflammatoire, cytostatique, cytotoxique, anti-prolifératif, anti-microtubules, anti-angiogénique, anti-resténotique (anti-resténose), anti-fongicide, anti-néoplasique, anti-migrative, athrombogénique et / ou anti-thrombogénique.

**11.** Le support vasculaire biodégradable selon une quelconque des revendications précédentes, où le revêtement polymérique exterieur contient au moins un agent actif anti-inflammatoire, cytostatique, cytotoxique, anti-prolifératif, anti-microtubules, anti-angiogénique, anti-resténotique (anti-resténose), anti-fongicide, anti-néoplasique, antimigrative, athrombogénique et / ou antithrombogénique

**12.** Le support vasculaire biodégradable selon la revendication 10 ou 11, dans lequel l'au moins un agent actif anti-inflammatoire, cytostatique, cytotoxique, anti-prolifératif, anti-microtubules, anti-angiogénique, anti-resténotique (anti-resténose), anti-fongicide, anti-néoplasique, antimigrative, athrombogénique et / ou antithrombogénique est sélectionné à partir du groupe comprenant:

abciximab, acemétacine, acétylvismione B, aclarubicine, ademétionine, adriamycine, aescin, afromosone, akagerine, aldesleukine, amidorone, aminoglutéthimide, amsacrine, anakinra, anastrozole, anémonin, anopterine, antimycotiques, antithrombotiques, apocymarine, argatroban, aristolactame-AII, acide aristolochique, ascomycine, asparaginase, aspirine, atorvastatine, auranofine, azathioprine, azithromycine, baccatin, bafilomycine, basiliximab, bendamustine, benzocaine, berbérine, bétuline, acide bétulinique, bilobol, bisparthenolidine, bléomycine, bombrestatin, acides boswelliques et leurs dérivés, brucéanol A, B et C, bryophylline A, busulfan, antithrombine, bivalirudine, cadhérines, camptothécine, capécitabine, acide o-carbamoyl-phenoxyacétique, carboplatine, carmustine, célécoxib, cépharanthine, cérivastatine, inhibiteurs de la CETP, chlorambucil, chloroquine phosphate, cictoxine, ciprofloxacine, cisplatine, cladribine, clarithromycine, colchicine, concanamycine, coumadin, peptide natriurétique de type C (CNP), cudraisoflavone A, curcumine, cyclophosphamide, ciclosporine A, cytarabine, dacarbazine, daclizumab, dactinomycin, dapsone, daunorubicine, diclofenac, 1,11-diméthoxycanthin-6-one, docétaxel, doxorubicine, daunomycine, épirubicine, épothilone A et B, érythromycine, éstramustine, étoboside, everolimus, filgrastim, fluroblastin, fluvastatin, fludarabine, fludarabine-5'-dihydrogéne phosphate, fluorouracile, folimycine, fosfestrol, gemcitabine, ghalakinoside, ginkgol, acide ginkgolique, glycoside 1a, 4-hydroxyoxycyclo phosphamide, idarubicine, ifosfamide, josamycine, lapachol, lomustine, lovastatin,

melphalan, midécamycine, mitoxantrone, nimustine, pitavastatine, pravastatine, procarbazine, mitomycine, méthotrexate, mércaptopurine, thioguanine, oxaliplatin, irinotécan, topotécan, hydroxycarbamide, miltéfosine, pentostatine, pegaspargase, exémestane, létrozole, formestane, inhibiteur de la prolifération de cellules des muscles lisses-2w, mitoxanthrone, mycophénolate mofétil, b-myc antisense, β-lapachone, podophyllotoxine, acide podophyllique-2-éthylhydrazide, molgramostim (rhuGM-CSF), pegintérferon α-2b, lenograstim (r-HuG-CSF), macrogol, sélectine (cytokine antagoniste), inhibiteurs des cytokines, inhibiteur de la COX-2, NFkB, angiopeptine, anticorps monoclonaux qui inhibitent la prolifération des cellules musculaires, antagonistes du bFGF, probucol, prostaglandines, 1-hydroxy-11-méthoxycanthin-6-one, scopolétol, donneurs de NO comme le pentaérythrityl tetranitrate et les sydnoimines, dérivés S-nitroso, tamoxifène, staurosporine, β-estradiol, α-estradiol, estriol, estrone, éthinyl estradiol, médroxyprogestérone, cypionates d'estradiol, benzoates d'estradiol, tranilast, kamebakaurine et autres terpénoides utilisés dans la thérapie du cancer, verapamil, inhibiteurs de la tyrosine kinase (tyrphostins), paclitaxel et ses dérivés comme 6-α-hydroxy-paclitaxel, taxotères, suboxydes du carbone et leurs oligomères macrocycliques, mofebutazone, lonazolac, lidocaine, kétoprofène, acide méfénamique, piroxicam, méloxicam, pénicillamine, hydroxychloroquine, aurothiomalate de sodium, oxacéprol, β-sitostérol, myrtécaïne, polidocanol, nonivamide, lévomenthol, ellipticine, D-24851 (Calbiochem), colcémid, cytochalasines A-E, indanocine, nocodazole, protéine S100, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des virus transmetteurs, fragments de l'ADN et l'ARN, inhibiteur-1 de l'activateur du plasminogène, inhibiteur-2 de l'activateur du plasminogène, les oligonucléotides antisens, les inhibiteurs du VEGF, l'IGF-1, agents actifs du groupe des antibiotiques comme céfadroxil, céfazolin, céfaclor, céfoxitin, tobramycine, gentamicine, pénicillines comme dicloxacilline, oxacilline, sulfonamides, métronidazole, énoxaparin, héparines desulfatées et N-réacétylés, activateur rtissulaire du plasminogène, recepteur membranaire de la plaquette GpIIb/IIIa, anticorps de l'inhibiteur du facteur Xa, héparine, hirudine, r-hirudine, PPACK, protamine, prourokinase, streptokinase, warfarine, urokinase, vasodilatateurs comme dipyramidole, trapidil, nitroprussides, antagonistes du PDGF comme triazolopyrimidine et seramin, inhibiteurs de l'ACE comme captopril, cilazapril, lisinopril, énalapril, losartan, inhibiteurs de la thioprotéase, prostacycline, vapiprost, interféron α, β et γ, antagonistes d'histamine, bloqueurs de la sérotonine, inhibiteurs de l'apoptose, régulateurs de l'apoptose comme p65, NF-kB ou des oligonucléotides antisense Bcl-xL, halofuginone, nifédipine, tocophérol, tranilast, molsidomine, polyphénols du thé, gallate d'épicatéchine, gallate d'épigallocatéchine, léflunomide, étanercept, sulfasalazine, etoposid, dicloxacylline, tétracycline, triamcinolone, mutamycine, procainimide, acide rétinoïque, quinidine, disopyrimide, flécaïnide, propafénone, sotolol, stéroïdes naturels et synthétiques comme inotodiol, maquiroside A, ghalakinoside, mansonine, strebloside, hydrocortisone, bétaméthasone, déxaméthasone, substances non-stéroïdiennes (NSAIDS) comme fénoprofène, ibuprofène, indomèthacine, naproxen, phénylbutazone et d'autres agents antiviraux comme acyclovir, ganciclovir et zidovudine, clotrimazole, flucytosine, griséofulvine, kétoconazole, miconazole, nystatin, terbinafine, agents antiprotozoaires comme chloroquine, méfloquine, quinine, d'autres terpénoïdes naturels comme hippocaesculine, barringtogénol-C21-angelate, 14-déhydroagrostistachine, agroskerine, agrostistachine, 17-hydroxyagrostistachine, ovatodiolids, acide 4,7-oxycycloanisomelique, baccharinoids B1, B2, B3 et B7, tubeimoside, bruceantinoside C, yadanziosides N et P, isodéoxyelephantopine, tomenphantopine A et B, coronarine A, B; C et D, acide ursolique, acide hyptatique A, iso-iridogermanal, maytenfoliol, effusantine A, excisanine A et B, longikaurine B, sculponeatin C, kamebaunin, leukamenin A et B, 13,18-déhydro-6-alpha-senecioyloxychaparrin, taxamairin A et B, regenilol, triptolide, cymarin, hydroxyanoptérine, protoanémonin, chlorure de cheliburin, sinococuline A et B, dihydronitidine, chlorure de nitidine, 12-β-hydroxypregnadièn-3,20-dione, hélénaline, indicine, indicine-N-oxyde, lasiocarpine, inotodiol, podophyllotoxin, justicidine A et B, larréatin, malloterin, mallotochromanol, isobutyrylmallotochromanol, maquiroside A, marchantine A, maytansine, lycoridicine, margétine, pancratistatin, liriodènine, bisparthenolidine, oxoushinsunine, periplocoside A, acide ursolique, déoxypsorospermine, psychorubine, ricin A, sanguinarine, acide du blé manwu, méthylsorbifoline, chromones de spathelia, stizophylline, mansonine, fasudil, streblosid, dihydro usambaraensine, hydroxyusambarine, strychnopentamine, strychnophylline, usambarine, usambarensine, liriodenine, oxoushinsunine, daphnoretine, laricirésinol, méthoxylaricirésinol, syringarésinol, sirolimus (rapamycine), somatostatine, tacrolimus, roxythromycine, troleadomycin, simvastatine, rosuvastatine, vinblastine, vincristine, vindesine, teniposide, vinorelbine, trofosfamide, tréosulfan, trémozolomide, thiotépa, trétinoine, spiramycine, umbellifèrone, desacétylvismione A, vismione A et B, zeorin.

**13.** Le support vasculaire biodégradable selon une quelconque des revendications précédentes, où le support vasculaire biodégradable est un stent pour les vaisseaux sanguins, les voies urinaires, les voies respiratoires, l'oesophage, les voies biliaires ou l'appareil digestif.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1419793 B1
- DE 10207161 A1
- DE 19856983 A1
- EP 0966979 A2
- WO 2007005806 A


### In der Beschreibung aufgeführte Nicht-Patentliteratur


- **SAKARIASSEN et al.** *J Lab- Clin. Med.,* 1983, vol. 102 (4), 522